# EUROPEAN PATENT SPECIFICATION

(11) **EP 4 231 986 B1**
(45) Date of publication and mention of the grant of the patent: **09.10.2024**
(21) Application number: 21791424.1
(22) Date of filing: 21.10.2021
(51) Int. Cl.: A61K 8/06, A61K 8/44, A61K 8/73, A61K 8/81, A61Q 1/00, A61Q 17/04, A61Q 19/00

(54) **PHOTOPROTECTIVE COMPOSITION**
LICHTSCHUTZZUSAMMENSETZUNG
COMPOSITION PHOTOPROTECTRICE

(30) Priority: 23.10.2020 FR 2010914
(43) Date of publication of application: 30.08.2023
(73) Proprietor: L'OREAL, 75008 Paris (FR)
(72) Inventor: DOUEZAN, Stéphane, 94152 CHEVILLY LARUE (FR); CATALAN-MARTIN, Elena, 94152 CHEVILLY LARUE (FR); NICOT, Ondine, 94152 CHEVILLY LARUE (FR); JOSSO, Martin, 94152 CHEVILLY LARUE (FR); BEAUDOUIN, Roselyne, 94152 CHEVILLY LARUE (FR); MERCIER, Céline, 94152 CHEVILLY LARUE (FR)
(74) Representative: Cabinet Nony
(86) International application number: PCT/EP2021/079237
(87) International publication number: WO 2022/084458

(56) References cited:
- EP-A1- 3 616 679
- WO-A1-2014/128679
- WO-A1-2016/030839
- FR-A1- 3 025 095
- FR-A1- 3 025 103

## Description

The present invention is directed toward proposing for the cosmetic field, and in particular for caring for, for the hygiene of, for protecting and/or for making up keratin materials, preferably for caring for the skin of the body or of the face, a new composition which is quite particularly advantageous with regard to its technical performance levels and the sensory feelings that it gives the user when applied thereto, in particular to the skin.

The term "keratin materials" is intended to mean in particular the skin, the lips, and also keratin fibres such as the hair, in particular the skin and/or the hair, and preferably the skin, in particular of the body and/or the face.

The present invention relates in particular to the field of photoprotective cosmetic compositions, also referred to as antisun products or photoprotective products, and also to the corresponding non-therapeutic cosmetic treatment processes.

### Technical field

Keratin materials, and in particular the skin, are exposed daily to sunlight.

As it happens, it is known that prolonged exposure of the skin to this polychromatic light is capable of inducing skin disorders or else superficial damage.

Due to this exposure, some people will especially see on the skin, and more specifically on the face, the neckline, the arms, the hands and the shoulders, the appearance of darker and/or more coloured marks, giving the skin a lack of uniformity, which for obvious reasons poses aesthetic problems.

To counter these undesirable effects, it is common practice to use photoprotective compositions in which organic or inorganic anti-UV screening agents are formulated.

They generally contain organic UV-screening agents and/or inorganic UV-screening agents, which function according to their own chemical nature and according to their own properties by absorption, reflection or scattering of the UV radiation. They generally contain mixtures of liposoluble organic screening agents and/or water-soluble UV-screening agents.

UVA-screening agents advantageously make it possible to screen UVA radiation, with wavelengths between 320 and 400 nm, which causes tanning of the skin and is liable to induce adverse changes therein, especially in the case of sensitive skin or skin that is continually exposed to solar radiation. UVA radiation causes in particular a loss of elasticity of the skin and the appearance of wrinkles, thereby leading to premature ageing of the skin. As for UVB-screening agents, they make it possible to screen UVB radiation, with wavelengths between 280 and 320 nm, which also enables tanning of the epidermis but is liable to cause erythemas and superficial skin burns which may be harmful to natural tanning.

### Prior art

A wide variety of photoprotective compositions are already known to date for protecting keratin materials, and more particularly the skin, against the harmful effects induced by UVA and/or UVB radiation. They mostly contain a combination of several organic or inorganic UV-screening agents, conveyed in an oily phase and/or in an aqueous phase as anti-UV active agent and are generally proposed in a presentation form of emulsion or gel type.

It is also known that high contents of screening agents are required to achieve high levels of screening efficiency.

However, high contents of UV-screening agents do not lend themselves to easy production of compositions having a stabilized and pleasant texture.

Thus, formulations with high screening power generally have uncomfortable or even unpleasant sensory aspects masking the freshness and comfort of the formulations. In particular, the weak point of photoprotective formulations with a high protection factor is often a strong greasy and tacky feel, and thus a lack of lightness and/or of freshness of the textures obtained, but also a white appearance on application, thus not being invisible on the skin.

Moreover, the introduction of a high content of UV-screening agents generally brings about destabilization problems. This instability may even occasionally cause phase separation and/or a loss of viscosity of the composition, making the formulation inefficient or even unusable.

To overcome the abovementioned undesirable effects, and in particular to obtain a fresh effect on application and an invisible effect on the skin, aqueous presentation forms have already been considered. However, these aqueous compositions containing UV-screening agents are generally tacky and thus uncomfortable, but also very fluid.

It has also been proposed to structure an aqueous gel containing screening agents using polymers. However, this solution is not satisfactory since the structuring polymers generally selected unfortunately exhibit low resistance to electrolytes and also degrade the sensory properties of the compositions.

In order to overcome the abovementioned undesirable effects, consideration has already been given to the use of aqueous compositions containing UV-screening agents in combination with other specific compounds.

However, these compositions lack freshness on application and exhibit stability defects over time.

### Disclosure of the invention

Consequently, it remains difficult to reconcile, in one and the same photoprotective composition, opposing technical performance qualities, such as a high level of UV protection, which usually implies a greasy and tacky finish on the skin, and a pleasant sensory aspect, brought about in particular by a fresh sensation.

There is still a need for a photoprotective composition with a high level of UV protection which is moreover transparent on application.

In particular, there is still a need for a photoprotective composition with a high level of UV protection, which has pleasant sensory properties and which is advantageously refreshing, and preferably transparent on application.

There is also a need for a photoprotective composition with a high level of UV protection, which is perfectly stable, i.e. not subject to demixing.

There therefore remains in particular a need to have a fresh, stable cosmetic composition which is transparent on application to keratin materials, which is neither greasy nor tacky, and which has good efficiency against solar radiation, in particular UV radiation, and notably a satisfactory sun protection factor (SPF).

The present invention is specifically directed towards meeting these needs.

### Summary of the invention

Following considerable research carried out on the question, it has now been found by the applicant, entirely unexpectedly and surprisingly, that it is possible to achieve these objectives by combining specific gelling agents.

Thus, according to a first of its aspects, the present invention relates to a composition, notably a cosmetic composition, in particular for making up and/or caring for keratin materials, comprising:
- at least one aqueous phase containing:
   (i) at least a first hydrophilic gelling agent chosen from non-starchy polysaccharides; and
   (ii) at least a second hydrophilic gelling agent chosen from 2-acrylamido-2-methylpropanesulfonic acid polymers and copolymers;
- at least one oily phase containing:
   (a) at least a first lipophilic gelling agent chosen from amino acid-based gelling agents; and
   (b) at least a second lipophilic gelling agent chosen from dextrin esters; and
- said composition also comprising at least one UV-screening agent.

Preferably, in a composition according to the invention, the aqueous and oily phases are gelled.

Thus, according to one preferred embodiment, the present invention relates to a composition, notably a cosmetic composition, in particular for making up and/or caring for keratin materials, comprising:
- at least one aqueous phase gelled with:
   (i) at least a first hydrophilic gelling agent chosen from non-starchy polysaccharides; and
   (ii) at least a second hydrophilic gelling agent chosen from 2-acrylamido-2-methylpropanesulfonic acid polymers and copolymers;
- at least one oily phase gelled with:
   (a) at least a first lipophilic gelling agent chosen from amino acid-based gelling agents; and
   (b) at least a second lipophilic gelling agent chosen from dextrin esters; and
- said composition also comprising at least one UV-screening agent.

Against all expectations, the inventors have observed that the combining of specific gelling agents with UV-screening agents in a formulation according to the invention makes it possible to obtain compositions which on application provide a sensation of great freshness while at the same time preserving good cosmetic properties, and a high level of protection. As emerges from the examples below, the compositions according to the invention exhibit a good freshness effect and are transparent on application.

They exhibit good protective properties and are stable, while at the same time preserving the freshness and transparency on application to keratin materials.

In addition, they advantageously withstand the addition of electrolytes and a large amount of alcohol, in particular of ethanol.

Preferably, the composition according to the invention has an architecture of gel/gel type. Thus, preferably, the composition according to the invention is different from an emulsion. According to one preferred embodiment, the compositions according to the invention do not comprise surfactants.

According to another of its aspects, a subject of the invention is also a non-therapeutic cosmetic process for making up and/or caring for a keratin material, in particular the skin and/or the lips, comprising at least one step which consists in applying to said keratin material a composition as defined above.

### Detailed description

### HYDROPHILIC GELLING AGENTS

For the purposes of the present invention, the term "hydrophilic gelling agent" means a compound that is capable of gelling the aqueous phase of the compositions according to the invention.

The hydrophilic gelling agents are thus present in the aqueous phase of the composition.

As specified above, the composition according to the invention comprises at least a first hydrophilic gelling agent (i) chosen from non-starchy polysaccharides, and at least a second hydrophilic gelling agent (ii) chosen from 2-acrylamido-2-methylpropanesulfonic acid polymers and copolymers.

### First hydrophilic gelling agent (i)

The composition according to the invention comprises at least a first hydrophilic gelling agent (i) chosen from non-starchy polysaccharides.

The non-starchy polysaccharides may be chosen from polysaccharides produced by microorganisms, polysaccharides isolated from algae, and higher plant polysaccharides, such as homogeneous polysaccharides, in particular celluloses and derivatives thereof or fructosans, heterogeneous polysaccharides such as gum arabic, galactomannans, glucomannans and pectins, and derivatives thereof; and mixtures thereof.

In particular, the non-starchy polysaccharides may be chosen from fructans, gellans, glucans, amylose, amylopectin, glycogen, pullulan, dextrans, celluloses and derivatives thereof, in particular methylcelluloses, hydroxyalkylcelluloses, ethylhydroxyethylcelluloses and carboxymethylcelluloses, mannans, xylans, lignins, arabans, galactans, galacturonans, alginate-based compounds, chitin, chitosans, glucuronoxylans, arabinoxylans, xyloglucans, glucomannans, pectic acids and pectins, arabinogalactans, carrageenans, agars, glycosaminoglucans, gum Arabic, tragacanth gums, ghatti gums, karaya gums, locust bean gums, galactomannans such as guar gums and non-ionic derivatives thereof, in particular hydroxypropyl guar, and ionic derivatives thereof, biopolysaccharide gums of microbial origin, in particular scleroglucan or xanthan gums, mucopolysaccharides, and in particular chondroitin sulfates, and mixtures thereof.

More particularly, the first hydrophilic gelling agent (i) may be chosen from carrageenans, in particular kappa carrageenan, gellan gum, agar-agar, xanthan gum, alginate-based compounds, in particular sodium alginate, scleroglucan gum, guar gum, inulin and pullulan, and mixtures thereof.

Preferably, the first hydrophilic gelling agent (i) is xanthan gum.

Preferably, the aqueous phase of a composition according to the invention comprises from 0.05% to 5% by weight, preferably from 0.1% to 1% by weight, of at least a first hydrophilic gelling agent (i), relative to the total weight of the composition.

### Second hydrophilic gelling agent (ii)

The composition according to the invention comprises at least a second hydrophilic gelling agent (ii) chosen from 2-acrylamido-2-methylpropanesulfonic acid polymers and copolymers.

Thus, the polymers used that are suitable as second hydrophilic gelling agent (ii) for the invention may be crosslinked or non-crosslinked homopolymers or copolymers including at least the 2-acrylamido-2-methylpropanesulfonic acid (AMPS^{®}) monomer, in a form partially or totally neutralized with a mineral base other than aqueous ammonia, such as sodium hydroxide or potassium hydroxide.

They are preferably totally or almost totally neutralized, i.e. at least 90% neutralized.

These AMPS^{®} polymers according to the invention may be crosslinked or non-crosslinked.

When the polymers are crosslinked, the crosslinking agents may be chosen from the polyolefinically unsaturated compounds commonly used for crosslinking polymers obtained by radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allyl or vinyl ethers of polyfunctional alcohols, and also the allylic esters of phosphoric and/or vinylphosphonic acid derivatives, or mixtures of these compounds.

According to a preferred embodiment of the invention, the crosslinking agent is chosen from methylenebisacrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The AMPS^{®} polymers that are suitable for use in the invention are water-soluble or water-dispersible. In this case, they are either "homopolymers" comprising only AMPS^{®} monomers and, if they are crosslinked, one or more crosslinking agents such as those defined above, or copolymers obtained from AMPS^{®} and one or more hydrophilic or hydrophobic ethylenically unsaturated monomers and, if they are crosslinked, one or more crosslinking agents such as those defined above. When said copolymers comprise hydrophobic ethylenically unsaturated monomers, these monomers do not comprise a fatty chain and are preferably present in small amounts.

For the purposes of the present invention, the term "fatty chain" means any hydrocarbon-based chain including at least 7 carbon atoms.

The term "water-soluble or water-dispersible" means polymers which, when introduced into an aqueous phase at 25°C, at a weight concentration equal to 1%, make it possible to obtain a macroscopically homogeneous and transparent solution, i.e. a solution with a maximum light transmittance value, at a wavelength equal to 500 nm, through a sample 1 cm thick, of at least 60% and preferably of at least 70%.

The "homopolymers" according to the invention are preferably crosslinked and neutralized, and they may be obtained according to the preparation process comprising the following steps: (a) the monomer such as AMPS^{®} is dispersed or dissolved in free form in a solution of tert-butanol or of water and tert-butanol; (b) the monomer solution or dispersion obtained in (a) is neutralized with one or more mineral or organic bases, preferably aqueous ammonia NH₃, in an amount making it possible to obtain a degree of neutralization of the sulfonic acid functions of the polymer ranging from 90% to 100%; (c) the crosslinking monomer(s) are added to the solution or dispersion obtained in (b); (d) a standard free-radical polymerization is performed in the presence of free-radical initiators at a temperature ranging from 10°C to 150°C; the polymer precipitating in the tert-butanol-based solution or dispersion.

The water-soluble or water-dispersible AMPS^{®} copolymers according to the invention contain water-soluble ethylenically unsaturated monomers, hydrophobic monomers, or mixtures thereof.

The water-soluble comonomers may be ionic or non-ionic.

Among the ionic water-soluble comonomers, mention may for example be made of the following compounds and salts thereof: (meth)acrylic acid, styrenesulfonic acid, vinylsulfonic acid and (meth)allylsulfonic acid, vinylphosphonic acid, maleic acid, itaconic acid, crotonic acid, water-soluble vinyl monomers of formula (A) below:

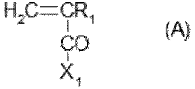

in which R₁ is chosen from H, -CH₃, -C₂H₅ or -C₃H₇; X₁ is chosen from alkyl oxides of type -OR₂ where R₂ is a linear or branched, saturated or unsaturated hydrocarbon-based radical containing from 1 to 6 carbon atoms, substituted with at least one sulfonic (-SO³-) and/or sulfate (-SO⁴-) and/or phosphate (-PO₄H₂-) group.

Among the non-ionic water-soluble comonomers, mention may for example be made of (meth)acrylamide, N-vinylacetamide and N-methyl N-vinylacetamide, N-vinylformamide and N-methyl-N-vinylformamide, maleic anhydride, vinylamine, N-vinyllactams comprising a cyclic alkyl group having from 4 to 9 carbon atoms, such as N-vinylpyrrolidone, N-butyrolactam and N-vinylcaprolactam, the vinyl alcohol of formula CH₂=CHOH, the water-soluble vinyl monomers of formula (B) below: 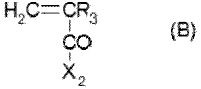 in which R₃ is chosen from H, -CH₃, -C₂H₅ or -C₃H₇; X₂ is chosen from alkyl oxides of the type -OR₄ where R₄ is a linear or branched, saturated or unsaturated hydrocarbon-based radical containing from 1 to 6 carbon atoms, optionally substituted with a halogen (iodine, bromine, chlorine or fluorine) atom; a hydroxyl (-OH) group; ether.

Mention is made, for example, of glycidyl (meth)acrylate, hydroxyethyl methacrylate, and (meth)acrylates of ethylene glycol, of diethylene glycol or of polyalkylene glycol.

Among the hydrophobic comonomers with no fatty chain, mention may for example be made of styrene and derivatives thereof such as 4-butylstyrene, alpha-methylstyrene and vinyltoluene; vinyl acetate of formula CH₂=CH-OCOCH₃; vinyl ethers of formula CH₂=CHOR in which R is a linear or branched, saturated or unsaturated, hydrocarbon-based radical having from 1 to 6 carbons; acrylonitrile; caprolactone; vinyl chloride and vinylidene chloride; silicone derivatives, resulting, after polymerization, in silicone polymers such as methacryloxypropyltris(trimethylsiloxy)silane and silicone methacrylamides; the hydrophobic vinyl monomers of formula (C) below: 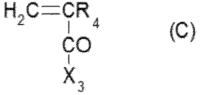 in which R₄ is chosen from H, -CH₃, -C₂H₅ or -C₃H₇; X₃ is chosen from alkyl oxides of the type -OR₅ where R₅ is a linear or branched, saturated or unsaturated hydrocarbon-based radical containing from 1 to 6 carbon atoms.

Mention is made, for example, of methyl methacrylate, ethyl methacrylate, n-butyl (meth)acrylate, tert-butyl (meth)acrylate, cyclohexyl acrylate, isobornyl acrylate and 2-ethylhexyl acrylate.

The water-soluble or water-dispersible AMPS^{®} polymers of the invention preferably have a molar mass ranging from 50 000 g/mol to 10 000 000 g/mol, preferably from 80 000 g/mol to 8 000 000 g/mol, and even more preferably from 100 000 g/mol to 7 000 000 g/mol.

As water-soluble or water-dispersible AMPS^{®} homopolymers suitable for use in the invention, mention may be made, for example, of crosslinked or non-crosslinked polymers of sodium acrylamido-2-methylpropanesulfonate, in particular that used in the commercial product Simulgel 800 (CTFA name: Sodium Polyacryloyldimethyl Taurate), crosslinked ammonium acrylamido-2-methylpropanesulfonate polymers (INCI name: Ammonium Polyacryldimethyltauramide) such as those described in patent EP 0 815 928 B 1 and such as the product sold under the trade name Hostacerin AMPS^{®} by the company Clariant.

Preferably, a composition according to the invention comprises an AMPS^{®} homopolymer.

As water-soluble or water-dispersible AMPS^{®} copolymers in accordance with the invention, examples that may be mentioned include:
- crosslinked acrylamide/sodium acrylamido-2-methylpropanesulfonate copolymers, in particular that used in the commercial product Sepigel 305 (CTFA name: Polyacrylamide/C₁₃-C₁₄ Isoparaffin/ Laureth-7) or that used in the commercial product sold under the name Simulgel 600 (CTFA: Acrylamide/sodium acryloyldimethyltaurate/isohexadecane/polysorbate-80) by the company SEPPIC;
- copolymers of AMPS^{®} and of vinylpyrrolidone or vinylformamide, in particular that used in the commercial product sold under the name Aristoflex AVC^{®} by the company Clariant (CTFA name: Ammonium Acryloyldimethyltaurate/VP copolymer) but neutralized with sodium hydroxide or potassium hydroxide;
- copolymers of AMPS^{®} and of sodium acrylate, in particular the AMPS^{®}/sodium acrylate copolymer, in particular that used in the commercial product sold under the name Simulgel EG^{®} by the company SEPPIC or under the trade name Sepinov EM (CTFA name: Hydroxyethyl Acrylate/Sodium Acryloyldimethyltaurate Copolymer);
- copolymers of AMPS^{®} and of hydroxyethyl acrylate, in particular the AMPS^{®}/hydroxyethyl acrylate copolymer, in particular that used in the commercial product sold under the name Simulgel NS^{®} by the company SEPPIC (CTFA name: Hydroxyethyl acrylate/sodium acryloyldimethyltaurate copolymer (and) squalane (and) polysorbate 60), or such as the product sold under the name sodium acrylamido-2-methylpropanesulfonate/hydroxyethyl acrylate copolymer, such as the commercial product Sepinov EMT 10 (INCI name: Hydroxyethyl acrylate/sodium acryloyldimethyltaurate copolymer).

As water-soluble or water dispersible AMPS^{®} copolymers which are preferred in accordance with the invention, mention may be made of copolymers of AMPS^{®} and of vinylpyrrolidone or vinylformamide, in particular that used in the commercial product sold under the name Aristoflex AVC^{®} by the company Clariant (CTFA name: Ammonium Acryloyldimethyltaurate/VP copolymer) but neutralized with sodium hydroxide or potassium hydroxide.

Thus, according to one preferred embodiment, the second hydrophilic gelling agent (ii) is chosen from 2-acrylamido-2-methylpropanesulfonic acid copolymers, preferably from copolymers of 2-acrylamido-2-methylpropanesulfonic acid and of vinylpyrrolidone or of vinylformamide, and more preferentially from copolymers of 2-acrylamido-2-methylpropanesulfonic acid and of vinylpyrrolidone.

Preferably, the aqueous phase of a composition according to the invention comprises from 0.1% to 5% by weight, preferably from 0.4% to 2% by weight, of at least a second hydrophilic gelling agent (ii), relative to the total weight of the composition.

### LIPOPHILIC GELLING AGENTS

For the purposes of the present invention, the term "lipophilic gelling agent" means a compound that is capable of gelling the oily phase of the compositions according to the invention.

The lipophilic gelling agents are thus present in the oily phase of the composition.

As specified above, the composition according to the invention comprises at least a first lipophilic gelling agent (a) chosen from amino acid-based gelling agents, and at least a second lipophilic gelling agent (b) chosen from dextrin esters.

It may likewise also comprise at least a third lipophilic gelling agent (c), in particular chosen from semi-crystalline polymers.

### First lipophilic gelling agent (a)

The composition according to the invention comprises at least a first lipophilic gelling agent (a) chosen from amino acid-based gelling agents.

In particular, the amino acid-based gelling agents may be chosen from N-acylamino acid derivatives, as defined in patent US 5843194, and notably from N-acylamino acid derivatives chosen from N-lauroylglutamic acid diethylamide, N-lauroylglutamic acid dibutylamide (dibutyl lauroyl glutamide), N-lauroylglutamic acid dihexylamide, N-lauroylglutamic acid dioctylamide, N-lauroylglutamic acid didecylamide, N-lauroylglutamic acid didodecylamide, N-lauroylglutamic acid ditetradecylamide, N-lauroylglutamic acid dihexadecylamide, N-lauroylglutamic acid distearylamide, N-stearoylglutamic acid dibutylamide, N-stearoylglutamic acid dihexylamide, N-stearoylglutamic acid dihexylamide (dibutyl ethylhexanoyl glutamide), N-stearoylglutamic acid diheptylamide, N-stearoylglutamic acid dioctylamide, N-stearoylglutamic acid didecylamide, N-stearoylglutamic acid didodecylamide, N-stearoylglutamic acid dioctylamide, N-stearoylglutamic acid dihexadecylamide, N-stearoylglutylamide, N-stearoylglutamic acid distearylamide, and mixtures thereof.

Preferably, the first lipophilic gelling agent (a) is chosen from dibutyl lauroyl glutamide and dibutyl ethylhexanoyl glutamide.

More preferentially, the first lipophilic gelling agent (a) is dibutyl lauroyl glutamide.

Preferably, the oily phase of a composition according to the invention comprises from 0.05% to 5% by weight, preferably from 0.1% to 1% by weight, of at least a first lipophilic gelling agent (a), relative to the total weight of the composition.

### Second lipophilic gelling agent (b)

The composition according to the invention comprises at least a second lipophilic gelling agent (b) chosen from dextrin esters.

In particular, the composition comprises a second lipophilic gelling agent (b) chosen from esters of dextrin and of a fatty acid, in particular a C₁₂ to C₂₄ and preferably C₁₄ to C₁₈ fatty acid, or mixtures thereof.

More preferentially, the dextrin ester is an ester of dextrin and of a C₁₂-C₁₈ and in particular C₁₄-C₁₈ fatty acid.

According to one particularly preferred embodiment, the dextrin ester is chosen from dextrin myristate and/or dextrin palmitate.

According to one particular embodiment, the dextrin ester is dextrin myristate, notably such as that sold under the name Rheopearl MKL-2, by the company Chiba Flour Milling.

Particularly preferably, the dextrin ester is dextrin palmitate. This product may be chosen, for example, from those sold under the names Rheopearl TL^{®}, Rheopearl KL^{®} and Rheopearl^{®} KL2 by the company Chiba Flour Milling.

Preferably, the oily phase of a composition according to the invention comprises from 0.1% to 5% by weight, preferably from 0.4% to 2% by weight, of at least a second lipophilic gelling agent (b), relative to the total weight of the composition.

### Third lipophilic gelling agent (c)

According to one preferred embodiment, the composition according to the invention may also comprise at least a third lipophilic gelling agent (c).

The third lipophilic gelling agent (c) may be in particular chosen from semi-crystalline polymers.

Preferably, the semi-crystalline polymer has an organic structure, and a melting point of greater than or equal to 30°C.

For the purposes of the invention, the term *"semi-crystalline polymer"* means polymers including a crystallizable portion and an amorphous portion and having a first-order reversible change of phase temperature, in particular of melting (solid-liquid transition). The crystallizable portion is either a side chain (or pendent chain) or a block in the backbone.

When the crystallizable part of the semi-crystalline polymer is a block of the polymer backbone, this crystallizable block has a chemical nature different from that of the amorphous blocks; in this case, the semi-crystalline polymer is a block copolymer, for example of the diblock, triblock or multiblock type. When the crystallizable portion is a chain that is pendent relative to the backbone, the semi-crystalline polymer may be a homopolymer or a copolymer.

The melting point of the semi-crystalline polymer is preferably less than 150°C.

The melting point of the semi-crystalline polymer is preferably greater than or equal to 30°C and less than 100°C. More preferably, the melting point of the semi-crystalline polymer is greater than or equal to 30°C and less than 70°C.

The semi-crystalline polymer(s) according to the invention are solid at ambient temperature (25°C) and atmospheric pressure (760 mmHg), with a melting point of greater than or equal to 30°C. The melting point values correspond to the melting point measured using a differential scanning calorimeter (DSC), such as the calorimeter sold under the name DSC 30 by the company Mettler, with a temperature rise of 5 or 10°C per minute (the melting point under consideration is the point corresponding to the temperature of the most endothermic peak in the thermogram).

The semi-crystalline polymer(s) according to the invention preferably have a melting point that is higher than the temperature of the keratin support intended to receive said composition, in particular the skin, the lips or the eyebrows.

According to the invention, the semi-crystalline polymers are advantageously soluble in the fatty phase, notably to at least 1% by weight, at a temperature that is higher than their melting point. Besides the crystallizable chains or blocks, the blocks of the polymers are amorphous. For the purposes of the invention, the term *"crystallizable chain or block"* means a chain or block which, if it were alone, would change from the amorphous state to the crystalline state reversibly, depending on whether the temperature is above or below the melting point. For the purposes of the invention, a chain is a group of atoms, which is pendent or lateral relative to the polymer backbone. A "block" is a group of atoms belonging to the backbone, this group constituting one of the repeating units of the polymer.

Preferably, the polymer backbone of the semi-crystalline polymers is soluble in the fatty phase at a temperature above their melting point.

Preferably, the crystallizable blocks or chains of the semi-crystalline polymers represent at least 30% of the total weight of each polymer and better still at least 40%. The semi-crystalline polymers bearing crystallizable side chains are homopolymers or copolymers. The semi-crystalline polymers of the invention bearing crystallizable blocks are block or multiblock copolymers. They may be obtained by polymerizing a monomer bearing reactive (or ethylenic) double bonds or by polycondensation. When the polymers of the invention are polymers bearing crystallizable side chains, these polymers are advantageously in random or statistical form.

Preferably, the semi-crystalline polymers of the invention are of synthetic origin.

According to a preferred embodiment, the semi-crystalline polymer is chosen from:
- homopolymers and copolymers including units resulting from the polymerization of one or more monomers bearing crystallizable hydrophobic side chain(s),
- polymers bearing in the backbone at least one crystallizable block,
- polycondensates of aliphatic or aromatic or aliphatic/aromatic polyester type,
- copolymers of ethylene and propylene prepared via metallocene catalysis, and
- acrylate/silicone copolymers.

The semi-crystalline polymers which can be used in the invention may be chosen in particular from:
- block copolymers of polyolefins of controlled crystallization, the monomers of which are described in EP 0 951 897,
- polycondensates, in particular of aliphatic or aromatic or aliphatic/aromatic polyester type,
- copolymers of ethylene and propylene prepared via metallocene catalysis,
- homopolymers or copolymers bearing at least one crystallizable side chain and homopolymers or copolymers bearing in the backbone at least one crystallizable block, such as those described in US-5 156 911, in particular the (C₁₀-C₃₀)alkyl polyacrylates corresponding to the Intelimer^{®} products from the company Landec described in the brochure Intelimer^{®} *Polymers,* Landec IP22 (Rev. 4-97), for example the product Intelimer^{®} IPA 13-1 from the company Landec, which is a stearyl polyacrylate with a molecular weight of about 145 000 and a melting point equal to 49°C, or the Tego^{®} products from the company Evonik, and for example the Tego^{®} SP 13-1 product, which is a stearyl polyacrylate with a molecular weight of about 145 000 and a melting point equal to 49°C,
- homopolymers or copolymers bearing at least one crystallizable side chain, in particular containing fluoro group(s), such as described in WO 01/19333,
- acrylate/silicone copolymers, such as copolymers of acrylic acid and of stearyl acrylate bearing polydimethylsiloxane grafts, copolymers of stearyl methacrylate bearing polydimethylsiloxane grafts, copolymers of acrylic acid and of stearyl methacrylate bearing polydimethylsiloxane grafts, copolymers of methyl methacrylate, butyl methacrylate, 2-ethylhexyl acrylate and stearyl methacrylate bearing polydimethylsiloxane grafts. Mention may be made in particular of the copolymers sold by the company Shin Etsu under the names KP-561 (CTFA name: Acrylates/Dimethicone), KP-541 (CTFA name: Acrylates/Dimethicone and Isopropyl Alcohol), KP-545 (CTFA name: Acrylates/Dimethicone and Cyclopentasiloxane), or KSG-16 (Dimethicone/Vinyl Dimethicone Crosspolymer (and) Dimethicone);
- and mixtures thereof.

Preferably, the third lipophilic gelling agent (c) is chosen from homopolymers or copolymers bearing at least one crystallizable side chain and homopolymers or copolymers bearing at least one crystallizable block in the backbone.

According to one particularly preferred embodiment, the third lipophilic gelling agent (c) is chosen from (C₁₀-C₃₀)alkyl polyacrylates, and more preferentially chosen from stearyl polyacrylates.

Preferably, the oily phase of a composition according to the invention comprises from 0.2% to 5% by weight, preferably from 0.5% to 2% by weight, of at least a third lipophilic gelling agent (c), relative to the total weight of the composition.

### UV-screening agents

As mentioned above, a composition according to the invention comprises at least one UV-screening agent.

In particular, a composition according to the invention may comprise at least one compound for screening out UVA and/or UVB.

Preferably, it may contain at least one UV-screening agent chosen from hydrophilic organic UV-screening agents, lipophilic organic UV-screening agents, insoluble organic UV-screening agents, mineral screening agents, and mixtures thereof.

According to one preferred embodiment, the composition, preferably a cosmetic composition, according to the invention may comprise at least one organic and/or mineral UV-screening agent (agent which screens out the UV radiation of sunlight).

The UV-screening agent is a UV-screening agent normally used in cosmetics. It may be chosen from the positive list contained in Annex VI of Regulation (EC) No 1223/2009, which specifies the list of UV-screening agents permitted in cosmetics.

The UV-screening agents of the composition according to the invention may be of various nature.

They may be lipophilic, hydrophilic or insoluble organic agents.

The term "lipophilic UV-screening agent" means any cosmetic or dermatological screening agent that can be fully dissolved in molecular form in a liquid fatty phase or that can be dissolved in colloidal form (for example in micellar form) in a liquid fatty phase.

The term "hydrophilic UV-screening agent" means any cosmetic or dermatological screening agent that can be fully dissolved in molecular form in a liquid aqueous phase or that can be dissolved in colloidal form (for example in micellar form) in a liquid aqueous phase.

The term "insoluble UV-screening agent" means any cosmetic or dermatological screening agent which is not defined either as a lipophilic UV-screening agent or as a hydrophilic UV-screening agent, and which is in the form of particles in aqueous phase or liquid fatty phase. The UV-screening agents of the composition according to the invention may afford UVA and/or UVB photoprotection.

According to one particular embodiment, the composition also comprises at least one UV-screening agent chosen from hydrophilic organic UV-screening agents, lipophilic organic UV-screening agents, insoluble organic UV-screening agents, mineral screening agents, and any mixture thereof.

According to one preferred embodiment, the composition also comprises at least one UV-screening agent chosen from hydrophilic organic UV-screening agents, lipophilic organic UV-screening agents, insoluble organic UV-screening agents, and any mixture thereof.

In particular, the composition may comprise one or more bis-resorcinyl triazine derivatives as described and prepared according to the syntheses indicated in patent application EP 0 775 698.

As examples of such compounds which can be used, mention may be made of:
- 2,4-bis{[4-(2-ethylhexyloxy)-2-hydroxy]phenyl}-6-(4-methoxyphenyl)-1,3,5-triazine;
- 2,4-bis{[4-(3-(2-propyloxy)-2-hydroxypropyloxy]-2-hydroxy]phenyl}-6-(4-methoxyphenyl)-1,3,5-triazine;
- 2,4-bis{[4-(2-ethylhexyloxy)-2-hydroxy]phenyl}-6-[4-(2-methoxyethylcarboxyl)phenylamino]-1,3,5-triazine;
- 2,4-bis{[4-tris(trimethylsiloxysilylpropyloxy)-2-hydroxy]phenyl}-6-(4-methoxyphenyl)-1,3,5-triazine;
- 2,4-bis{[4-(2"-methylpropenyloxy)-2-hydroxy]phenyl}-6-(4-methoxyphenyl)-1,3,5-triazine;
- 2,4-bis{[4-(1',1',1',3',5',5',5'-heptamethyltrisiloxy-2"-methylpropyloxy)-2-hydroxylphenyll-6-(4-methoxyphenyl)-1,3,5-triazine;
- 2,4-bis{[4-(3-(2-propyloxy)-2-hydroxypropyloxy]-2-hydroxy]phenyl}-6-[(4-ethylcarboxyl)phenylamino]-1,3,5-triazine;
- 2,4-bis{[4-(2-ethylhexyloxy)-2-hydroxy]phenyl}-6-(1-methylpyrrol-2-yl)-1,3,5-triazine. Use may be made more particularly of at least the compound 2,4-bis{[4-(2-ethylhexyloxy)-2-hydroxy]phenyl}-6-(4-methoxyphenyl)-1,3,5-triazine or Bis-Ethylhexyloxyphenol Methoxyphenyl Triazine (INCI name), in particular the product sold under the trade name Tinosorb S by Ciba-Geigy.

According to one embodiment, the composition may comprise one or more dibenzoylmethane derivatives. Mention may be made especially, in a nonlimiting manner, of:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

According to one embodiment, the composition may comprise one or more benzylidenecamphor derivatives. Mention may be made especially of:
- 3-benzylidenecamphor, in particular produced under the name Mexoryl SD by Chimex,
- 4-methylbenzylidenecamphor, in particular sold under the name Eusolex 6300 by Merck,
- benzylidenecamphorsulfonic acid, in particular produced under the name Mexoryl SL by Chimex,
- camphor benzalkonium methosulfate, in particular produced under the name Mexoryl SO by Chimex,
- polyacrylamidomethylbenzylidenecamphor, in particular produced under the name Mexoryl SW by Chimex,
- terephthalylidenedicamphorsulfonic acid, in particular produced under the name Mexoryl SX by Chimex.

The organic UV-screening agents may also be chosen from anthranilics; cinnamic derivatives; salicylic derivatives; benzophenone derivatives; phenyl benzotriazole derivatives; benzalmalonate derivatives, notably those mentioned in patent US 5 624 663; phenyl benzimidazole derivatives; imidazolines; 4,4-diarylbutadiene derivatives; bisbenzazolyl derivatives, as described in patents EP 0 669 323 and US 2 463 264; p-aminobenzoic acid (PABA) derivatives; methylenebis(hydroxyphenylbenzotriazole) derivatives as described in applications US 5 237 071, US 5 166 355, GB 2 303 549, DE 197 26 184 and EP 893 119; benzoxazole derivatives, as described in applications EP 0 832 642, EP 1 027 883, EP 1300 137 and DE 101 62 844; screening polymers and screening silicones such as those described notably in application WO 93/04665; α-alkylstyrene-based dimers, such as those described in application DE 198 55 649; 4,4-diarylbutadienes as described in applications EP 0 967 200, DE 197 46 654, DE 197 55 649, EP-A-1 008 586, EP 1 133 980 and EP 133 981; other merocyanin derivatives such as those described in applications WO 04/006878, WO 05/058269 and WO 06/032741; and mixtures thereof.

As examples of organic UV-screening agents used in the composition according to the invention, mention may be made of those denoted below under their INCI name.

### Lipophilic UVA-screening agents:

### Dibenzoylmethane derivatives:

- Isopropyldibenzoylmethane;
- Butyl Methoxydibenzoylmethane (also known under the name Avobenzone).

### Aminobenzophenones:

- n-Hexyl 2-(4-diethylamino-2-hydroxybenzoyl)benzoate) (INCI name: diethylamino hydroxybenzoyl hexyl benzoate) sold notably under the trade name Uvinul A+ by BASF;
- 1,1'-(1,4-Piperazinediyl)bis[1-[2-[4-(diethylamino)-2-hydroxybenzoyl]phenyl]methanone (CAS 919803-06-8).

### Anthranilic derivatives:

- Menthyl anthranilate, sold especially under the trade name Neo Heliopan MA by Symrise. *4,4-Diarylbutadiene derivatives:*
- 1,1-Dicarboxy(2,2'-dimethylpropyl)-4,4-diphenylbutadiene.

### Merocyanine derivatives:

- Octyl 5-N,N-diethylamino-2-phenylsulfonyl-2,4-pentadienoate.

According to one preferred embodiment, the composition according to the invention comprises Butyl methoxydibenzoylmethane.

### Hydrophilic UVA-screening agents:

The bis-benzazolyl derivatives as described in patents EP 669 323 and US 2 463 264 and more particularly the compound disodium phenyl dibenzimidazotetrasulfonate, notably sold under the trade name Neo Heliopan AP by Symrise.

According to a particular embodiment, the composition according to the invention comprises terephthalylidenedicamphorsulfonic acid (sold for example under the trade name Mexoryl SX).

### Lipophilic UVB-screening agents:

### para-Aminobenzoates:

- Ethyl PABA;
- Ethyl dihydroxypropyl PABA;
- Ethylhexyl dimethyl PABA (Escalol 507 from ISP).

### Salicylic derivatives:

- Homosalate, in particular sold under the name Eusolex HMS by RonalEM Industries;
- Ethylhexyl salicylate, in particular sold under the name Neo Heliopan OS by Symrise;
- Dipropylene glycol salicylate, in particular sold under the name Dipsal by Scher;
- TEA salicylate, in particular sold under the name Neo Heliopan TS by Symrise.

### Cinnamates:

- Ethylhexyl methoxycinnamate, in particular sold under the trade name Parsol MCX by DSM Nutritional Products, Inc.;
- Isopropyl methoxycinnamate;
- Isoamyl methoxycinnamate, in particular sold under the trade name Neo Heliopan E 1000 by Symrise;
- Diisopropyl methyl cinnamate;
- Cinnoxate;
- Glyceryl ethylhexanoate dimethoxycinnamate.

### β,β'-Diphenyl acrylate derivatives:

- Etocrylene, in particular sold under the trade name Uvinul N35 by BASF;
- Octocrylene, in particular sold under the trade name Uvinul N539 by BASF.

### Benzylidenecamphor derivatives:

- 3-Benzylidenecamphor, in particular sold under the name Mexoryl SD by Chimex;
- Methylbenzylidenecamphor, in particular sold under the name Eusolex 6300 by Merck;
- Polyacrylamidomethylbenzylidenecamphor, in particular sold under the name Mexoryl SW by Chimex.

### Triazine derivatives:

- Ethylhexyl triazone, in particular sold under the trade name Uvinul T150 by BASF;
- Diethylhexyl butamido triazone, in particular sold under the trade name Uvasorb HEB by Sigma 3V;
- 2,4,6-Tris(dineopentyl 4'-aminobenzalmalonate)-s-triazine;
- 2,4,6-Tris(diisobutyl 4'-aminobenzalmalonate)-s-triazine;
- 2,4-Bis(dineopentyl 4'-aminobenzalmalonate)-6-(n-butyl 4'-aminobenzoate)-s-triazine;
- 2,4-Bis(n-butyl 4'-aminobenzoate)-6-(aminopropyltrisiloxane)-s-triazine;
- the symmetrical triazine screening agents described in patent US 6 225 467, application WO 2004/085412 (see compounds 6 and 9) or the document Symmetrical Triazine Derivatives, IP.COM Journal, IP.COM INC West Henrietta, NY, US (20 September 2004), especially 2,4,6-tris(biphenyl)-1,3,5-triazines (in particular 2,4,6-tris(biphenyl-4-yl-1,3,5-triazine) and 2,4,6-tris(terphenyl)-1,3,5-triazine, the latter two screening agents being described in Beiersdorf applications WO 06/035000, WO 06/034982, WO 06/034991, WO 06/035007, WO 2006/034992 and WO 2006/034985).

### Imidazoline derivatives:

- Ethylhexyl dimethoxybenzylidene dioxoimidazoline propionate.

### Benzalmalonate derivatives:

- Polyorganosiloxanes containing benzalmalonate functions, in particular Polysilicone-15, sold notably under the trade name Parsol SLX by DSM Nutritional Products, Inc.;
- Dineopentyl 4'-methoxybenzalmalonate.

According to one preferred embodiment, the composition according to the invention comprises one or more UV-screening agents chosen from the following screening agents: Homosalate (in particular sold under the name Eusolex HMS by RonalEM Industries); Ethylhexyl salicylate (in particular sold under the name NEO Heliopan OS by Symrise); Octocrylene (in particular sold under the trade name Uvinul N539 by BASF); Ethylhexyl triazone (in particular sold under the trade name Uvinul T150), and any mixture thereof.

### Hydrophilic UVB-screening agents:

The following p-aminobenzoic acid (PABA) derivatives:
- PABA;
- Glyceryl PABA; and
- PEG-25 PABA, in particular sold under the trade name Uvinul P25 by BASF;
- Phenylbenzimidazolesulfonic acid, in particular sold under the trade name Eusolex 232 by Merck;
- ferulic acid;
- salicylic acid;
- DEA methoxycinnamate;
- Benzylidenecamphorsulfonic acid, in particular sold under the name Mexoryl SL by Chimex;
- Camphor benzalkonium methosulfate, in particular sold under the name Mexoryl SO by Chimex.

### Lipophilic mixed UVA and UVB screening agents:

### Benzophenone derivatives:

- Benzophenone-1, in particular sold under the trade name Uvinul 400 by BASF;
- Benzophenone-2, in particular sold under the trade name Uvinul D50 by BASF;
- Benzophenone-3 or Oxybenzone, in particular sold under the trade name Uvinul M40 by BASF;
- Benzophenone-6, in particular sold under the trade name Helisorb 11 by Norquay;
- Benzophenone-8, in particular sold under the trade name Spectra-Sorb UV-24 by American Cyanamid;
- Benzophenone-10;
- Benzophenone-11;
- Benzophenone-12.

### Phenylbenzotriazole derivatives:

- Drometrizole trisiloxane, in particular sold under the name Silatrizole by Rhodia Chimie or under the name Meroxyl XL by the company Chimex;
- Methylenebis(benzotriazolyl)tetramethylbutylphenol, in particular sold in solid form notably under the trade name Mixxim BB/100 by Fairmount Chemical, or in micronized form as an aqueous dispersion notably under the trade name Tinosorb M by Ciba Specialty Chemicals.

According to one preferred embodiment, the composition according to the invention comprises Drometrizole Trisiloxane (in particular under the name Silatrizole by Rhodia Chimie or under the name Meroxyl XL by the company Chimex).

### Benzoxazole derivatives:

- 2,4-Bis[5-1(dimethylpropyl)benzoxazol-2-yl-(4-phenyl)imino]-6-(2-ethylhexyl)imino-1,3,5-triazine, in particular under the name Uvasorb K2A by Sigma 3V.

### Hydrophilic mixed UVA and UVB screening agents:

Benzophenone derivatives comprising at least one sulfonic radical, in particular:
- Benzophenone-4 sold notably under the trade name Uvinul 40 by BASF;
- Benzophenone-5; and
- Benzophenone-9.

### UV-screening agents of the merocyanine family:

The composition, preferably cosmetic composition, according to the invention may comprise one or more UV-screening agents of the merocyanine family. Mention may also be made of the merocyanine-type screening agents in particular prepared according to the protocols described in WO 2007/071582, in IP.com Journal (2009), 9(5A), 29-30 IPCOM000182396D under the title Process for producing 3-amino-2-cyclohexan-1-ylidene compounds and in US 4 749 643 (column 13, line 66 - column 14, line 57 and the references cited in this regard). According to one preferred embodiment, the composition comprises one or more lipophilic UV-screening agents.

According to one preferred embodiment, the composition comprises one or more UV-screening agent(s) chosen from lipophilic UVA-screening agents, lipophilic UVB-screening agents and/or lipophilic mixed UVA and UVB screening agents.

More preferentially, the composition comprises one or more UV-screening agent(s) chosen from dibenzoylmethane derivatives, salicylic derivatives, 0,0'-diphenyl acrylate derivatives, triazine derivatives and phenylbenzotriazole derivatives.

According to one particularly preferred embodiment, the composition comprises one or more UV-screening agent(s) chosen from Butyl Methoxydibenzoylmethane, Homosalate, Ethylhexyl Salicylate, Octocrylene, Ethylhexyl triazone, Drometrizole Trisiloxane, and mixtures thereof.

According to one embodiment, the amount of the non-encapsulated organic UV-screening agent(s) present in the composition according to the invention may range from 0.5% to 50% by weight, relative to the total weight of the composition. It ranges for example from 5% to 50% by weight, or else for example from 10% to 40% by weight, and even for example ranges from 15% to 35% by weight, relative to the total weight of the composition. According to one particular embodiment, the concentration of UV-screening agents in the composition according to the invention ranges from 1% to 50% and preferably from 1% to 40% by weight, relative to the total weight of the composition.

The composition of the invention may also comprise mineral UV-screening agents, which are generally pigments. The pigments may or may not be coated.

The coated pigments are pigments that have undergone one or more surface treatments of chemical, electronic, mechanochemical and/or mechanical nature with compounds as described, for example, in Cosmetics & Toiletries, February 1990, Vol. 105, pages 53-64, such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

As is known, silicones are organosilicon polymers or oligomers comprising a linear or cyclic and branched or crosslinked structure, of variable molecular weight, obtained by polymerization and/or polycondensation of suitably functionalized silanes and essentially constituted of a repetition of main units in which the silicon atoms are connected to each other via oxygen atoms (siloxane bond), optionally substituted hydrocarbon-based radicals being connected directly to said silicon atoms via a carbon atom.

The term "silicones" also encompasses the silanes required for their preparation, in particular alkylsilanes.

The silicones used for coating the pigments that are suitable for the present invention are preferably chosen from the group containing alkylsilanes, polydialkylsiloxanes and polyalkylhydrogenosiloxanes. Even more preferentially, the silicones are chosen from the group containing octyltrimethylsilane, polydimethylsiloxanes and polymethylhydrogenosiloxanes.

Needless to say, before being treated with silicones, the metal oxide pigments may have been treated with other surface agents, in particular with cerium oxide, alumina, silica, aluminum compounds or silicon compounds, or mixtures thereof.

The coated pigments are, for example, titanium oxides that have been coated:
- with silica, the product Sunveil from the company Ikeda and the product Eusolex T-Avo from the company Merck,
- with silica and iron oxide, in particular the product Sunveil F from from the company Ikeda,
- with silica and alumina, in particular the products Microtitanium Dioxide MT 500 SA and Microtitanium Dioxide MT 100 SA from the company Tayca, Tioveil from the company Tioxide and Mirasun TiW 60 from the company Rhodia,
- with alumina, in particular the products Tipaque TTO-55 (B) and Tipaque TTO-55 (A) from the company Ishihara and UVT 14/4 from the company Kemira,
- with alumina and aluminum stearate, in particular the product Microtitanium Dioxide MT 100 TV, MT 100 TX, MT 100 Z and MT-01 from the company Tayca, and the products Solaveil CT-10 W, Solaveil CT 100 and Solaveil CT 200 from the company Uniqema,
- with silica, alumina and alginic acid, in particular the product MT-100 AQ from the company Tayca,
- with alumina and aluminum laurate, in particular the product Microtitanium Dioxide MT 100 S from the company Tayca,
- with iron oxide and iron stearate, in particular the product Microtitanium Dioxide MT 100 F from the company Tayca,
- with zinc oxide and with zinc stearate, in particular the product BR351 from the company Tayca,
- with silica and alumina and treated with a silicone, in particular the products Microtitanium Dioxide MT 600 SAS, Microtitanium Dioxide MT 500 SAS or Microtitanium Dioxide MT 100 SAS from the company Tayca,
- with silica, alumina and aluminum stearate and treated with a silicone, in particular the product STT-30-DS from the company Titan Kogyo,
- with silica and treated with a silicone, in particular the product UV-Titan X 195 from the company Kemira or the product SMT-100 WRS from the company Tayca,
- with alumina and treated with a silicone, in particular the products Tipaque TTO-55 (S) from the company Ishihara or UV Titan M 262 from the company Kemira,
- with triethanolamine, in particular the product STT-65-S from the company Titan Kogyo,
- with stearic acid, in particular the product Tipaque TTO-55 (C) from the company Ishihara,
- with sodium hexametaphosphate, in particular the product Microtitanium Dioxide MT 150 W from the company Tayca.

Other titanium oxide pigments treated with a silicone are, for example, TiO2 treated with octyltrimethylsilane, such as the product sold under the trade name T 805 by the company Degussa Silices, TiO2 treated with a polydimethylsiloxane, such as the product sold under the trade name 70250 Cardre UF TiO2SI3 by the company Cardre, anatase/rutile TiO2 treated with a polydimethylhydrogenosiloxane, such as the product sold under the trade name Microtitanium Dioxide USP Grade Hydrophobic by the company Color Techniques. The uncoated titanium oxide pigments are sold, for example, by the company Tayca under the trade names Microtitanium Dioxide MT 500 B or Microtitanium Dioxide MT 600 B, by the company Degussa under the name P 25, by the company Wackher under the name Transparent titanium oxide PW, by the company Miyoshi Kasei under the name UFTR, by the company Tomen under the name ITS and by the company Tioxide under the name Tioveil AQ.

The uncoated zinc oxide pigments are, for example:
- those sold under the name Z-Cote by the company Sunsmart,
- those sold under the name Nanox by the company Elementis,
- those sold under the name Nanogard WCD 2025 by the company Nanophase Technologies.

The coated zinc oxide pigments are, for example:
- those sold under the name Z-Cote HP1, by the company Sunsmart (dimethicone-coated ZnO),
- those sold under the name Zinc Oxide CS-5 by the company Toshibi (polymethylhydrogenosiloxane-coated ZnO),
- those sold under the name Nanogard Zinc Oxide FN by the company Nanophase Technologies (as a 40% dispersion in Finsolv TN, C₁₂-C₁₅ alkyl benzoate),
- those sold under the names Daitopersion Zn-30 and Daitopersion Zn-50 by the company Daito (dispersions in oxyethylenated polydimethylsiloxane/cyclopolymethylsiloxane comprising 30% or 50% of zinc oxides coated with silica and polymethylhydrogenosiloxane),
- those sold under the name NFD Ultrafine ZnO by the company Daikin (ZnO coated with perfluoroalkyl phosphate and copolymer based on perfluoroalkylethyl as a dispersion in cyclopentasiloxane),
- those sold under the name SPD-Z1 by the company Shin-Etsu (ZnO coated with silicone-grafted acrylic polymer, dispersed in cyclodimethylsiloxane),
- those sold under the name Escalol Z100 by the company ISP (alumina-treated ZnO dispersed in an ethylhexyl methoxycinnamate/PVP-hexadecene copolymer/methicone mixture),
- those sold under the name Fuji ZnO-SMS-10 by the company Fuji Pigment (ZnO coated with silica and polymethylsilsesquioxane),
- those sold under the name Nanox Gel TN by the company Elementis (ZnO dispersed at a concentration of 55% in C₁₂-C₁₅ alkyl benzoate with hydroxystearic acid polycondensate). The uncoated cerium oxide pigments are sold, for example, under the name Colloidal Cerium Oxide.

The uncoated iron oxide pigments are sold, for example, by the company Arnaud under the names Nanogard WCD 2002 (FE 45B), Nanogard Iron FE 45 BL AQ, Nanogard FE 45R AQ and Nanogard WCD 2006 (FE 45R) or by the company Mitsubishi under the name TY-220.

The coated iron oxide pigments are sold, for example, by the company Arnaud under the names Nanogard WCD 2008 (FE 45B FN), Nanogard WCD 2009 (FE 45B 556), Nanogard FE 45 BL 345 and Nanogard FE 45 BL or by the company BASF under the name Transparent Iron Oxide.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A, and also the mixture of titanium dioxide and zinc dioxide coated with alumina, silica and silicone, such as the product M 261 sold by the company Kemira, or coated with alumina, silica and glycerol, such as the product M 211 sold by the company Kemira.

The pigments may be introduced into the compositions according to the invention in their native form or in the form of a pigmentary paste, i.e. as a mixture with a dispersant, as described, for example, in document GB 2 206 339.

According to one particular embodiment, the composition according to the invention does not comprise mineral UV-screening agents.

According to one particular embodiment, the amount of the mineral UV-screening agent(s) present in the composition according to the invention may range from 0.01% to 20% by weight, relative to the total weight of the composition. It ranges, for example, from 1% to 15% by weight, relative to the total weight of the composition.

According to a particular embodiment, the composition according to the invention also comprises one or more organic UV-screening agents and one or more mineral UV-screening agents.

According to one particular embodiment, the composition according to the invention also comprises a combination of UV-screening agents as described in patent FR 2 977 490, application WO 2013/004777 or application US 2014/0134120.

Preferably, in a composition according to the invention, the UV-screening agent(s) are all or partly, and preferably solely, present in the oily phase, preferably in the gelled oily phase.

### AQUEOUS PHASE

The aqueous phase of a composition according to the invention comprises water and optionally a water-soluble solvent.

Preferably, the aqueous phase of a composition according to the invention is gelled.

The aqueous phase may be present in the composition in a content ranging from 20% to 95% by weight, better still from 30% to 90% by weight and preferably from 50% to 80% by weight relative to the total weight of said composition.

Preferably, a composition according to the invention comprises from 35% to 70% by weight, preferably from 35% to 65% by weight and more preferentially from 40% to 60% by weight of water, relative to the total weight of the composition.

A water that is suitable for use in the invention may be a floral water such as cornflower water and/or a mineral water such as Vittel water, Lucas water or La Roche Posay water and/or a thermal spring water.

In the present invention, the term "water-soluble solvent" denotes a compound that is liquid at ambient temperature and water-miscible (miscibility in water of greater than 50% by weight at 25°C and atmospheric pressure).

The water-soluble solvents which can be used in the composition of the invention can in addition be volatile.

Mention may in particular be made, among the water-soluble solvents which can be used in the composition in accordance with the invention, of lower monoalcohols having from 1 to 5 carbon atoms, such as ethanol and isopropanol, glycols having from 2 to 8 carbon atoms, such as ethylene glycol, propylene glycol, 1,3-butylene glycol and dipropylene glycol, C₃ and C₄ ketones and C₂-C₄ aldehydes.

According to one preferred embodiment, the composition according to the invention also comprises at least one alcohol, in particular a monoalcohol, and preferably ethanol. Preferably, in a composition according to the invention, the alcohol may be present in a content ranging from 0.1% to 10% by weight and preferably ranging from 2% to 7% by weight, relative to the total weight of the composition.

According to an alternative embodiment, the aqueous phase of a composition according to the invention may comprise at least one C₂-C₃₂ polyol.

For the purposes of the present invention, the term *"polyol"* should be understood as meaning any organic molecule including at least two free hydroxyl groups.

Preferably, a polyol in accordance with the present invention is present in liquid form at ambient temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are advantageously suitable for formulating a composition according to the present invention are those especially containing from 2 to 32 carbon atoms and preferably 3 to 16 carbon atoms.

Advantageously, the polyol may be chosen, for example, from ethylene glycol, pentaerythritol, trimethylolpropane, propylene glycol, 1,3-propanediol, 1,2-pentanediol, caprylyl glycol (1,2-octanediol), butylene glycol, isoprene glycol, pentylene glycol, hexylene glycol, glycerol, polyglycerols, such as glycerol oligomers, for instance diglycerol, and polyethylene glycols, and mixtures thereof.

According to one particular mode, the composition of the invention may comprise at least one 1,2-pentanediol.

According to one particular mode, the composition of the invention may comprise at least caprylyl glycol (1,2-octanediol).

According to a particular mode, the composition of the invention may comprise at least glycerol.

According to one preferred embodiment of the invention, said polyol is chosen from caprylyl glycol 1,2-pentanediol and glycerol, and mixtures thereof.

### FATTY PHASE

A composition according to the invention comprises at least one fatty phase, preferably at least one gelled fatty phase.

In particular, a composition according to the invention comprises from 5% to 80% by weight, preferably from 10% to 70% by weight and more preferentially from 15% to 40% by weight of fatty phase, relative to the total weight of the composition.

The fatty phase of the composition according to the invention may comprise oils, waxes, pasty compounds, and/or silicone compounds, and preferably at least one oil.

### Oils

The term "oil" is intended to mean a water-immiscible non-aqueous compound that is liquid at ambient temperature (20°C) and at atmospheric pressure (760 mmHg).

An oily phase that is suitable for preparing the compositions, notably cosmetic compositions, according to the invention may comprise hydrocarbon-based oils, silicone oils, fluoro oils or non-fluoro oils, or mixtures thereof.

The oils may be volatile or non-volatile.

They may be of animal, plant, mineral or synthetic origin.

For the purposes of the present invention, the term " hydrocarbon-based oil" is intended to mean an oil mainly containing hydrogen and carbon atoms.

The term "silicone oil" is intended to mean an oil comprising at least one silicon atom and notably at least one Si-O group.

The term "fluoro oil" is intended to mean an oil comprising at least one fluorine atom.

The oils may optionally comprise oxygen, nitrogen, sulfur and/or phosphorus atoms, for example in the form of hydroxyl or acid radicals.

### Volatile oils

For the purposes of the invention, the term "volatile oil" is intended to mean any oil that is capable of evaporating on contact with the skin in less than one hour, at ambient temperature and atmospheric pressure. The volatile oil is a volatile cosmetic compound, which is liquid at ambient temperature, in particular having a non-zero vapour pressure, at ambient temperature and atmospheric pressure, in particular having a vapour pressure ranging from 0.13 Pa to 40 000 Pa (10⁻³ to 300 mmHg), in particular ranging from 1.3 Pa to 13 000 Pa (0.01 to 100 mmHg), and more particularly ranging from 1.3 Pa to 1300 Pa (0.01 to 10 mmHg).

The volatile oils may be hydrocarbon-based oils or silicone oils.

Among the volatile hydrocarbon-based oils containing from 8 to 16 carbon atoms, mention may be made notably of branched C₈-C₁₆ alkanes, for instance C₈-C₁₆ isoalkanes (also known as isoparaffins), isododecane, isodecane, isohexadecane and, for example, the oils sold under the trade names Isopar or Permethyl, branched C₈-C₁₆ esters, for instance isohexyl neopentanoate, and mixtures thereof. In particular, the volatile hydrocarbon-based oil is chosen from volatile hydrocarbon-based oils containing from 8 to 16 carbon atoms and mixtures thereof.

Mention may also be made of volatile linear alkanes comprising from 8 to 16 carbon atoms, in particular from 10 to 15 carbon atoms and more particularly from 11 to 13 carbon atoms, for instance n-dodecane (C₁₂) and n-tetradecane (C₁₄) sold by Sasol under the respective references Parafol 12-97 and Parafol 14-97, and also mixtures thereof, the undecane-tridecane mixture, mixtures of n-undecane (C₁₁) and of n-tridecane (C₁₃) obtained in examples 1 and 2 of application WO 2008/155 059 from the company Cognis, and mixtures thereof.

Volatile silicone oils that may be mentioned include volatile linear silicone oils such as hexamethyldisiloxane, octamethyltrisiloxane, decamethyltetrasiloxane, tetradecamethylhexasiloxane, hexadecamethylheptasiloxane and dodecamethylpentasiloxane.

Volatile cyclic silicone oils that may be mentioned include hexamethylcyclotrisiloxane, octamethylcyclotetrasiloxane, decamethylcyclopentasiloxane, cyclohexasiloxane and dodecamethylcyclohexasiloxane, and in particular cyclohexasiloxane.

### Non-volatile oils

The term "non-volatile" is intended to mean to an oil of which the vapour pressure at ambient temperature and atmospheric pressure is non-zero and is less than 10⁻³ mmHg (0.13 Pa). The non-volatile oils may notably be chosen from non-volatile hydrocarbon-based, fluoro and/or silicone oils.

Non-volatile hydrocarbon-based oils that may in particular be mentioned include:
- hydrocarbon-based oils of animal origin,
- hydrocarbon-based oils of plant origin, synthetic ethers containing from 10 to 40 carbon atoms, such as dicapryl ether,
- synthetic esters, for instance the oils of formula R₁COOR₂, in which R₁ represents a linear or branched fatty acid residue including from 1 to 40 carbon atoms and R₂ represents a hydrocarbon-based chain that is notably branched, containing from 1 to 40 carbon atoms, on condition that R₁ + R₂ is greater than or equal to 10. The esters may be chosen especially from alcohol and fatty acid esters, for instance cetostearyl octanoate, isopropyl alcohol esters, such as isopropyl myristate, isopropyl palmitate, ethyl palmitate, 2-ethylhexyl palmitate, isopropyl stearate, octyl stearate, hydroxylated esters, for instance isostearyl lactate, octyl hydroxystearate, alcohol or polyalcohol ricinoleates, hexyl laurate, neopentanoic acid esters, for instance isodecyl neopentanoate, isotridecyl neopentanoate, and isononanoic acid esters, for instance isononyl isononanoate and isotridecyl isononanoate,
- polyol esters and pentaerythritol esters, for instance dipentaerythrityl tetrahydroxystearate/tetraisostearate,
- fatty alcohols that are liquid at ambient temperature, with a branched and/or unsaturated carbon-based chain containing from 12 to 26 carbon atoms, for instance 2-octyldodecanol, isostearyl alcohol and oleyl alcohol,
- C₁₂-C₂₂ higher fatty acids, such as oleic acid, linoleic acid, linolenic acid, and mixtures thereof,
- carbonates, such as dicaprylyl carbonate,
- non-phenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, trimethylpentaphenyltrisiloxane, and mixtures thereof;
and also mixtures of these various oils.

In particular, the composition may also comprise at least one non-volatile oil, chosen in particular from non-volatile apolar hydrocarbon-based oils, non-volatile ester oils, and mixtures thereof.

For the purposes of the present invention, the term "apolar oil" means an oil of which the solubility parameter at 25°C, δₐ, is equal to 0 (J/cm³)^{½}.

The definition and calculation of the solubility parameters in the Hansen three-dimensional solubility space are described in the article by C. M. Hansen: "The three dimensional solubility parameters", J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation δₐ = (δp² + δh²)^{½}.

The parameters δₚ, δₕ, δ_{D} and δₐ are expressed in (J/cm³)^{½}.

In particular, the non-volatile apolar hydrocarbon-based oil is free of oxygen atoms. Preferably, the non-volatile apolar hydrocarbon-based oil may be chosen from linear or branched hydrocarbons of mineral or synthetic origin. In particular, it may be chosen from:
- liquid paraffin or derivatives thereof,
- squalane,
- liquid petroleum jelly,
- naphthalene oil,
- polybutylenes, in particular Indopol H-100 (molar mass or Mw = 965 g/mol), Indopol H-300 (Mw = 1340 g/mol) and Indopol H-1500 (Mw = 2160 g/mol) sold or produced by the company Amoco,
- polyisobutenes and hydrogenated polyisobutenes, in particular Parleam^{®} sold by the company Nippon Oil Fats, Panalane H-300 E sold or produced by the company Amoco (Mw = 1340 g/mol), Viseal 20000 sold or produced by the company Synteal (Mw = 6000 g/mol) and Rewopal PIB 1000 sold or produced by the company Witco (Mw = 1000 g/mol),
- decene/butene copolymers and polybutene/polyisobutene copolymers, in particular Indopol L-14,
- polydecenes and hydrogenated polydecenes, in particular Puresyn 10 (Mw = 723 g/mol) and Puresyn 150 (Mw = 9200 g/mol) sold or produced by the company Mobil Chemicals,
- and mixtures thereof.

Said non-volatile oil may also be an ester oil, in particular containing between 18 and 70 carbon atoms.

Examples that may be mentioned include monoesters, diesters or triesters.

The ester oils may notably be hydroxylated.

The non-volatile ester oil may preferably be chosen from:
- monoesters comprising between 18 and 40 carbon atoms in total, in particular the monoesters of formula R₁COOR₂ in which R₁ represents a linear or branched fatty acid residue including from 4 to 40 carbon atoms and R₂ represents a hydrocarbon-based chain that is in particular branched, containing from 4 to 40 carbon atoms, on condition that R₁ + R₂ is greater than or equal to 18, for instance Purcellin oil (cetostearyl octanoate), isononyl isononanoate, C₁₂ to C₁₅ alcohol benzoate, 2-ethylhexyl palmitate, octyldodecyl neopentanoate, 2-octyldodecyl stearate, 2-octyldodecyl erucate, isostearyl isostearate, diisopropyl sebacate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate or 2-diethylhexyl succinate. Preferably, they are esters of formula R₁COOR₂ in which R₁ represents a linear or branched fatty acid residue including from 4 to 40 carbon atoms and R₂ represents a hydrocarbon-based chain that is in particular branched, containing from 4 to 40 carbon atoms, R₁ and R₂ being such that R₁ + R₂ is greater than or equal to 18. Preferably, the ester comprises between 18 and 40 carbon atoms in total. Preferred monoesters that may be mentioned include isononyl isononanoate, oleyl erucate and/or 2-octyldodecyl neopentanoate;
- diesters, in particular comprising between 18 and 60 carbon atoms in total, in particular between 18 and 50 carbon atoms in total. It is in particular possible to use diesters of dicarboxylic acids and of monoalcohols, preferably such as diisostearyl malate, or glycol diesters of monocarboxylic acids, such as neopentyl glycol diheptanoate or polyglyceryl-2 diisostearate, in particular such as the compound sold under the trade reference Dermol DGDIS by the company Alzo;

- triesters, in particular comprising between 35 and 70 carbon atoms in total, in particular such as triesters of tricarboxylic acids, such as triisostearyl citrate, or tridecyl trimellitate, or glycol triesters of monocarboxylic acids such as polyglyceryl-2 triisostearate;
- tetraesters, in particular with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or else pentaerythrityl tetrakis(2-decyl)tetradecanoate;
- polyesters obtained by condensation of unsaturated fatty acid dimer and/or trimer and of diol, such as those described in patent application FR 0 853 634, in particular such as of dilinoleic acid and of 1,4-butanediol. Mention may notably be made in this respect of the polymer sold by Biosynthis under the name Viscoplast 14436H (INCI name: dilinoleic acid/butanediol copolymer), or else copolymers of polyols and of dimer diacids, and esters thereof, such as Hailuscent ISDA;
- esters and polyesters of diol dimer and of monocarboxylic or dicarboxylic acid, such as esters of diol dimer and of fatty acid and esters of diol dimer and of dicarboxylic acid dimer, in particular which may be obtained from a dicarboxylic acid dimer derived in particular from the dimerization of an unsaturated fatty acid, notably an unsaturated C₈ to C₃₄, notably C₁₂ to C₂₂, in particular C₁₆ to C₂₀ and more particularly C₁₈ fatty acid, such as esters of dilinoleic diacids and of dilinoleic diol dimers, for instance those sold by the company Nippon Fine Chemical under the trade names Lusplan DD-DA5^{®} and DD-DA7^{®};
- vinylpyrrolidone/1-hexadecene copolymers, for instance the product sold under the name Antaron V-216 (also known as Ganex V216) by the company ISP (Mw = 7300 g/mol);
- hydrocarbon-based plant oils such as fatty acid triglycerides (which are liquid at ambient temperature), in particular of fatty acids containing from 7 to 40 carbon atoms, such as heptanoic or octanoic acid triglycerides or jojoba oil; mention may be made in particular of saturated triglycerides such as caprylic/capric triglyceride, glyceryl triheptanoate, glyceryl trioctanoate, and C₁₈-C₃₆ acid triglycerides such as those sold under the reference DUB TGI 24 by Stearinerie Dubois; and unsaturated triglycerides such as castor oil, olive oil, ximenia oil and pracaxi oil;
- and mixtures thereof.

Preferably, a composition according to the invention comprises a non-volatile oil, in particular a non-volatile hydrocarbon-based oil, and more preferentially dicapryl ether.

### Silicone compounds

According to the present invention, the fatty phase may also comprise at least one silicone compound.

In particular, the silicone compound may be chosen from organopolysiloxane elastomers.

The term "organopolysiloxane elastomer" or "silicone elastomer" is intended to mean a flexible, deformable organopolysiloxane with viscoelastic properties and notably with the consistency of a sponge or a flexible sphere. Its modulus of elasticity is such that this material withstands deformation and has limited stretchability and contractility. This material is capable of regaining its original shape after stretching.

It is more particularly a crosslinked organopolysiloxane elastomer.

The elastomer is advantageously a non-emulsifying elastomer.

The term "non-emulsifying" defines organopolysiloxane elastomers not containing any hydrophilic chains, and in particular not containing any polyoxyalkylene units (notably polyoxyethylene or polyoxypropylene) or any polyglyceryl units. Thus, according to a specific form of the invention, the composition comprises an organopolysiloxane elastomer that is free of polyoxyalkylene units and of polyglyceryl unit.

In particular, the silicone elastomer used in the present invention is chosen from Dimethicone Crosspolymer (INCI name), Vinyl Dimethicone Crosspolymer (INCI name), Dimethicone/Vinyl Dimethicone Crosspolymer (INCI name), Dimethicone (and) Dimethicone/Vinyl Dimethicone Crosspolymer (INCI name) and Dimethicone Crosspolymer-3 (INCI name).

The organopolysiloxane elastomer particles may be conveyed in the form of a gel formed from an elastomeric organopolysiloxane included in at least one hydrocarbon-based oil and/or one silicone oil. In these gels, the organopolysiloxane particles are often non-spherical particles.

Non-emulsifying elastomers are especially described in patents EP242219, EP285886 and EP765656, and in application JP-A-61-194009.

The silicone elastomer is generally in the form of a gel, a paste or a powder, but advantageously in the form of a gel in which the silicone elastomer is dispersed in a linear silicone oil (dimethicone) or cyclic silicone oil (e.g.: cyclopentasiloxane), advantageously in a linear silicone oil.

Non-emulsifying elastomers which can be used more particularly include those sold under the names KSG-6, KSG-15, KSG-16, KSG-18, KSG-41, KSG-42, KSG-43 and KSG-44 by the company Shin-Etsu, DC9040 and DC9041 by the company Dow Corning, and SFE 839 by the company General Electric.

According to a particular mode, use is made of a gel of silicone elastomer dispersed in a silicone oil chosen from a non-exhaustive list comprising cyclopentadimethylsiloxane, dimethicones, dimethylsiloxanes, methyl trimethicone, phenyl methicone, phenyl dimethicone, phenyl trimethicone and cyclomethicone, preferably a linear silicone oil chosen from polydimethylsiloxanes (PDMS) or dimethicones with a viscosity at 25°C ranging from 1 to 500 cSt, optionally modified with optionally fluorinated aliphatic groups, or with functional groups such as hydroxyl, thiol and/or amine groups.

Mention may notably be made of the compounds having the following INCI names:
- Dimethicone/Vinyl Dimethicone Crosspolymer, such as USG-105 and USG-107A from the company Shin-Etsu; DC9506 and DC9701 from the company Dow Corning;
- Dimethicone/Vinyl Dimethicone Crosspolymer (and) Dimethicone, such as KSG-6 and KSG-16 from the company Shin-Etsu;
- Dimethicone/Vinyl Dimethicone Crosspolymer (and) Cyclopentasiloxane, such as KSG-15;
- Cyclopentasiloxane (and) Dimethicone Crosspolymer, such as DC9040, DC9045 and DC5930 from the company Dow Corning;
- Dimethicone (and) Dimethicone Crosspolymer, such as DC9041 from the company Dow Corning;
- Dimethicone (and) Dimethicone Crosspolymer, such as Dow Corning EL-9240^{®} Silicone Elastomer Blend from the company Dow Corning (mixture of polydimethylsiloxane crosslinked with hexadiene/polydimethylsiloxane (2 cSt));
- C₄₋C₂₄ alkyl dimethicone/divinyl dimethicone crosspolymer, such as NuLastic Silk MA from the company Alzo.

As examples of silicone elastomers dispersed in a linear silicone oil that can be used preferably according to the invention, mention may notably be made of Dimethicone/Vinyl Dimethicone Crosspolymer (and) Dimethicone, in particular KSG-6 and KSG-16 from the company Shin Etsu.

The particles of organopolysiloxane elastomers can also be used in powder form. Mention may be made notably of the powders sold under the names Dow Corning 9505 Powder and Dow Corning 9506 Powder by the company Dow Corning, these powders having the INCI name: Dimethicone/Vinyl Dimethicone Crosspolymer.

The organopolysiloxane powder may also be coated with silsesquioxane resin, as described, for example, in patent US 5 538 793. Such elastomeric powders are sold under the names KSP-100, KSP-101, KSP-102, KSP-103, KSP-104 and KSP-105 by the company Shin-Etsu, and have the INCI name: Vinyl Dimethicone/Methicone Silsesquioxane Crosspolymer.

### ADJUVANTS

### Fillers

Advantageously, a composition according to the invention may also comprise one or more fillers, in particular chosen from those conventionally used in care and/or makeup compositions.

For the purposes of the present invention, the term "fillers" is understood to mean colourless or white, mineral or organic, natural or synthetic solid particles of any form, which are in a form that is insoluble and dispersed in the medium of the composition.

Of course, these fillers are used in appropriate contents and under appropriate conditions so as not to be detrimental to the properties of the composition.

These fillers make it possible to give the composition containing them softness, a matt effect and uniformity. In addition, these fillers advantageously make it possible to combat various attacking factors such as sebum or sweat.

By way of illustration of these fillers, mention may be made of talc, mica, silica, kaolin, poly-β-alanine powder and polyethylene powder, tetrafluoroethylene polymer powders (Teflon^{®}), lauroyllysine, starch, boron nitride, hollow polymer microspheres such as those of polyvinylidene chloride/acrylonitrile, for instance Expancel^{®} (Nobel Industrie), acrylic acid copolymer microspheres, silicone resin microbeads (for example Tospearls^{®} from Toshiba), polyorganosiloxane elastomer particles, precipitated calcium carbonate, magnesium carbonate, magnesium hydrogen carbonate, hydroxyapatite, barium sulfate, aluminium oxides, polyurethane powders, composite fillers, hollow silica microspheres, and glass or ceramic microcapsules. Use may also be made of particles that are in the form of hollow sphere portions, as described in patent applications JP-2003 128 788 and JP-2000 191 789.

### Colorants

A composition according to the invention may also comprise at least one particulate or non-particulate, water-soluble or water-insoluble colorant, preferably in a proportion of at least 0.0001% by weight relative to the total weight of the composition.

For obvious reasons, this amount is liable to vary significantly with regard to the intensity of the desired colour effect and of the colour intensity afforded by the colorants under consideration, and its adjustment clearly falls within the competence of those skilled in the art.

As stated above, the colorants that are suitable for use in the invention may be water-soluble, but may also be liposoluble.

### Water-soluble dyes

For the purposes of the invention, the term "water-soluble colorant" is intended to mean any natural or synthetic, generally organic compound, which is soluble in an aqueous phase or water-miscible solvents and which is capable of imparting colour.

As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophyllin, methylene blue, anthocyanins (enocyanin, black carrot, hibiscus and elder), caramel and riboflavin.

The water-soluble dyes are, for example, beetroot juice and caramel.

According to a preferred embodiment, the composition according to the invention comprises at least one water-soluble dye.

The water-soluble dyes may be present in a proportion of from 0.0001% to 2% by weight relative to the total weight of the composition containing them.

### Liposoluble dyes

For the purposes of the invention, the term "liposoluble colorant" is intended to mean any natural or synthetic, generally organic compound, which is soluble in an oily phase or in solvents that are miscible with a fatty substance, and which is capable of imparting colour. As liposoluble dyes that are suitable for use in the invention, mention may be made especially of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

The colouring particulate materials may be present in a proportion of from 0.0001% to 10% by weight relative to the total weight of the composition containing them.

They may especially be pigments, nacres and/or particles with metallic tints.

### Pigments

The term "pigments" should be understood as meaning white or coloured, mineral or organic particles that are insoluble in an aqueous solution, which are intended to colour and/or opacify the composition containing them.

The pigments may be white or coloured, and mineral and/or organic.

As mineral pigments, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.

They may also be pigments having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

They may also be pigments having a structure that may be, for example, of silica microsphere type containing iron oxide. An example of a pigment having this structure is that sold by the company Miyoshi under the reference PC Ball PC-LL-100 P, this pigment being constituted of silica microspheres containing yellow iron oxide.

Advantageously, the pigments may be iron oxides and/or titanium dioxides.

According to one particular embodiment, a composition according to the invention comprises less than 1% of pigments.

### Nacres

The term "nacres" should be understood as meaning coloured particles of any form, which may or may not be iridescent, especially produced by certain molluscs in their shell, or alternatively synthesized, and which have a colour effect via optical interference.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic colorants. Examples of nacres that may also be mentioned include natural mica covered with titanium oxide, with iron oxide, with natural pigment or with bismuth oxychloride.

Mention may be made, among the commercially available nacres, of the Timica, Flamenco and Duochrome (on a mica base) nacres sold by Engelhard, the Timiron nacres sold by Merck, the Prestige nacres on a mica base sold by Eckart and the Sunshine synthetic mica-based nacres sold by Sun Chemical.

The nacres may more particularly have a yellow, pink, red, bronze, orange, brown, gold and/or coppery colour or tint.

Advantageously, the nacres in accordance with the invention are micas covered with titanium dioxide or with iron oxide, and also bismuth oxychloride.

According to one particular embodiment, a composition according to the invention comprises less than 1% by weight of nacres.

### Particles with a metallic tint

For the purposes of the present invention, the term "particles with a metallic tint" means any compound of which the nature, size, structure and surface finish allow it to reflect incident light, in particular in a non-iridescent manner.

The particles with a metallic tint which can be used in the invention are in particular chosen from particles of at least one metal and/or at least one metal derivative; particles comprising an organic or mineral substrate, made from a single material or multiple materials, at least partially covered with at least one layer with a metallic tint comprising at least one metal and/or at least one metal derivative, and mixtures of said particles.

Among the metals that may be present in said particles, mention may for example be made of Ag, Au, Cu, Al, Ni, Sn, Mg, Cr, Mo, Ti, Zr, Pt, Va, Rb, W, Zn, Ge, Te, Se and mixtures or alloys thereof. Ag, Au, Cu, Al, Zn, Ni, Mo and Cr, and mixtures or alloys thereof (for example bronzes and brasses), are preferred metals.

The term "metal derivatives" denotes compounds derived from metals, in particular oxides, fluorides, chlorides and sulfides.

Illustrations of these particles that may be mentioned include aluminium particles, such as those sold under the names Starbrite 1200 EAC^{®} by the company Siberline and Metalure^{®} by the company Eckart and glass particles coated with a metallic layer, especially those described in documents JP-A-09188830, JP-A-10158450, JP-A-10158541, JP-A-07258460 and JP-A-05017710.

According to one particular embodiment, a composition according to the invention comprises less than 1% of particles with a metallic tint.

### Hydrophobic treatment of the colorants

The pulverulent colorants as described previously may be totally or partially surface treated, with a hydrophobic agent, to make them more compatible with the oily phase of the composition of the invention, especially so that they have good wettability with oils. Thus, these treated pigments are well dispersed in the oily phase. Hydrophobically treated pigments are described especially in document EP-A-1 086 683.

The hydrophobic-treatment agent may be chosen from silicones such as methicones, dimethicones and perfluoroalkylsilanes; fatty acids, such as stearic acid; metal soaps, such as aluminium dimyristate, the aluminium salt of hydrogenated tallow glutamate; perfluoroalkyl phosphates; polyhexafluoropropylene oxides; perfluoropolyethers; amino acids; N-acylamino acids or salts thereof; lecithin, isopropyl triisostearyl titanate, isostearyl sebacate, and mixtures thereof.

The term "alkyl" mentioned in the compounds cited above especially denotes an alkyl group having from 1 to 30 carbon atoms and preferably having from 5 to 16 carbon atoms.

### Additional cosmetic active agent

According to one preferred embodiment, a composition according to the invention also comprises at least one additional cosmetic active agent.

In particular, the additional cosmetic active agent may be at least one hydrophilic active agent and/or one lipophilic active agent.

The term "hydrophilic active agent" is intended to mean a water-soluble or water-dispersible active agent, capable of forming hydrogen bonds.

As cosmetic active agents, mention may be made for example of moisturizers, preferably glycerol, depigmenting agents, desquamating agents, humectants, anti-ageing agents, mattifying agents, cicatrizing agents, antibacterial agents, care agents, preferably panthenol, vitamins and derivatives thereof, preferably tocopherol, antioxidant compounds, and mixtures thereof.

The additional active agent(s) may notably be chosen from:
- vitamins and derivatives thereof, notably esters thereof, in particular tocopherol (vitamin E) and esters thereof (for instance tocopheryl acetate), and ascorbic acid (vitamin C) and derivatives thereof;
- humectants, in particular urea, hydroxyureas, glycerol, polyglycerols, glyceryl glucoside, diglyceryl glucoside, polyglyceryl glucosides and xylityl glucoside, and in particular glycerol;
- C-glycoside compounds;
- antioxidant compounds;
- anti-ageing active agents, in particular hyaluronic acid compounds, and notably sodium hyaluronate, retinol and derivatives thereof, salicylic acid compounds and in particular 5-n-octanoylsalicylic acid (capryloylsalicylic acid), caffeine, adenosine, c-β-d-xylopyranoside-2-hydroxypropane and the sodium salt of (3-hydroxy-2-pentylcyclopentyl)acetic acid;
- skincare agents, in particular allantoin, panthenol and protein hydrolysates;
- veinotonics, in particular polyphenols, and notably escin, ruscus, diosmin, hesperidin, resveratrol, and
- mixtures thereof.

A composition according to the invention may comprise at least one moisturizer (also known as a humectant).

Preferably, the moisturizer is glycerol.

According to one preferred embodiment, a composition according to the invention also comprises at least one additional cosmetic active agent, and more preferentially at least one anti-ageing active agent, preferably hyaluronic acid, or a salt thereof, and more preferentially sodium hyaluronate.

A composition according to the invention may also include at least one additive chosen from the adjuvants conventionally used in the cosmetic field, such as preserving agents, in particular phenoxyethanol, fragrances, polar additives, film-forming polymers, additional gelling agents, pH modifiers (acids or bases), dispersants, cosmetic active agents, for instance cicatrizing agents, agents for combating greasy skin, and/or anti-pollution agents, and mixtures thereof.

Needless to say, those skilled in the art will take care to select this or these optional additional compound(s), and/or the amount thereof, such that the advantageous properties of a composition according to the invention are not, or are not substantially, adversely affected by the envisioned addition.

### COMPOSITION

According to the invention, the composition can be characterized before or after its application to keratin materials. In other words, the appearance of a composition according to the invention may be different before application, in other words in its packing/packaging, or after application to keratin materials, that is to say as soon as it is applied to keratin materials, in particular the skin.

According to a preferred embodiment, the composition according to the invention is coloured before application. In other words, the composition according to the invention is preferably coloured in its packing/packaging. Preferably, before application, the composition according to the invention is blue.

For the purposes of the invention, a composition is said to be *"coloured"* when it appears, to the human eye, to have a colour of the visible spectrum, in particular a colour distinct from white. Such a composition may be opaque or transparent/translucent.

According to another preferred embodiment, the composition according to the invention is white before application. In other words, the composition according to the invention is preferably white in its packing/packaging.

According to one particular embodiment, the composition according to the invention is transparent/translucent before application. In other words, the composition according to the invention is preferably transparent/translucent in its packing/packaging and/or when it is taken out of its packing and before spreading.

According to one preferred embodiment, after application to keratin materials, the composition according to the invention is not coloured.

The transparency/translucency can be evaluated by measurement of the average free path of the 1* photons, carried out with a Turbiscan^{™} Lab Analyzer instrument from the company Formulaction. The average free path is expressed in mm. The higher this value, the more transparent the composition.

Thus, the composition according to the invention preferably has an average free photon path length ranging from 0.5 mm to 10 mm, and preferably ranging from 2 to 10 mm. Preferably, whether the composition is opaque or transparent/translucent, coloured or colourless, before application, it is transparent or translucent when it is applied to keratin materials, in particular the skin.

Thus, as soon as it is applied to keratin materials, the composition according to the invention is transparent or translucent. The transparency or translucency of the composition can also be assessed visually. It can be seen for example that the composition applied to the skin, for example with a thickness of 150 µm, is transparent, that is to say leaves the keratin materials, in particular the skin, visible.

This transparency on application can also be evaluated according to a representative in vitro protocol, by depositing a film 150 µm thick on a transparent film of polyester, and by measuring its transmission with a Hazegard instrument from the company BYK-Gardner. The higher this value, the more transparent the composition.

Thus, the composition according to the invention preferably has a transmission of greater than 70% and preferably greater than 85%.

A composition according to the invention may be in the form of an emulsion, for example an oil-in-water (O/W) or water-in-oil (W/O) emulsion, of a gel, for example an oil-in-water or water-in-oil emulsified gel, or else in the form of a composition of gel/gel type.

Preferably, a composition according to the invention is of gel/gel type.

Thus, according to this embodiment, a composition according to the invention is different from an emulsion, or else different from an emulsified gel.

An emulsion is generally constituted of an oily liquid phase and an aqueous liquid phase. It is a dispersion of droplets of one of the two liquid phases in the other. The size of the droplets forming the dispersed phase of the emulsion is typically about a micrometre (0.1 to 100 µm). Furthermore, an emulsion requires the presence of a surfactant or of an emulsifier to ensure its stability over time.

Conversely, a composition of gel/gel type is constituted of a mixture of two immiscible gelled phases, both having a texture of gel type. This texture is notably reflected visually by a consistent and/or creamy appearance.

More specifically, in a composition of gel/gel type, the gelled aqueous phase and the gelled oily phase interpenetrate and thus form a stable, consistent product. This consistency is achieved by mixing interpenetrated macrodomains. These interpenetrated macrodomains are not measurable objects. Thus, by microscope, the composition of gel/gel type is very different from an emulsion. A composition of gel/gel type also cannot be characterized as having a "sense", i.e. an O/W or W/O sense.

Thus, a composition of gel/gel type has a consistency of gel type. The stability of the composition is long-lasting without surfactant. Consequently, a composition, notably a cosmetic composition, of gel/gel type does not require any surfactant or silicone emulsifier to ensure its stability over time.

It is known practice from the prior art to observe the intrinsic nature of a mixture of aqueous and oily gels in a composition of gel-gel type, for example, by introducing a colorant either into the aqueous gelled phase or into the lipophilic gelled phase, before the formation of the composition of gel-gel type. During visual inspection, in a composition of gel-gel type, the colorant appears uniformly dispersed, even if the dye is present solely in the gelled aqueous phase or in the gelled oily phase. Specifically, if two different dyes of different colours are introduced, respectively, into the oily phase and into the aqueous phase, before formation of the composition of gel-gel type, the two colours may be observed as being uniformly dispersed throughout the composition of gel-gel type. This is different from an emulsion in which, if a dye, which is soluble in water or soluble in oil, is introduced, respectively, into the aqueous and oily phases, before forming the emulsion, only the colour of the dye present in the outer phase will be observed (Remington: The Science and Practice of Pharmacy, 19th Edition (1995), Chapter 21, page 282).

In the case of the present invention, the test that will be preferred for distinguishing a composition of gel-gel type from an emulsion is a dilution test. Specifically, in a composition of gel-gel type, the aqueous and oily gelled domains interpenetrate and form a consistent and stable composition, in which the behaviour in water and in oil is different from the behaviour of an emulsion. Consequently, the behaviour during dilution of a gel-type composition (bi-continuous system) may be compared to that of an emulsion.

More specifically, the dilution test consists in placing 40 g of product and 160 g of dilution solvent (water or oil) in a 500 ml plastic beaker. The dilution is performed with controlled stirring to avoid any emulsification. In particular, this is performed using a planetary mixer: Speed Mixer TM DAC400FVZ. The speed of the mixer is set at 1500 rpm for 4 minutes. Finally, observation of the resulting sample is performed using a light microscope at a magnification of ×100 (×10×10). It may be noted that oils such as Parleam^{®} and Xiameter PMX-200 Silicone Fluid 5CS^{®} sold by Dow Corning are suitable as dilution solvent, in the same way as one of the oils contained in the composition.

In the case of a composition of gel-gel type (bi-continuous system), when it is diluted in oil or in water, a heterogeneous appearance is always observed. When a composition of gel-gel type (bi-continuous system) is diluted in water, pieces of oily gel in suspension are observed, and when a composition of gel-gel type (bi-continuous system) is diluted in oil, pieces of aqueous gel in suspension are observed.

In contrast, during dilution, emulsions have a different behaviour. When an O/W emulsion is diluted in an aqueous solvent, it gradually reduces without having a heterogeneous and lumpy appearance. This same O/W emulsion, on dilution with oil, has a heterogeneous appearance (pieces of O/W emulsion suspended in the oil). When a W/O emulsion is diluted with an aqueous solvent, it has a heterogeneous appearance (pieces of W/O emulsion suspended in the water). This same W/O emulsion, when diluted in oil, gradually reduces without having a heterogeneous and lumpy appearance.

Another test which may particularly be preferred for distinguishing a composition of gel/gel type from an emulsion or from an emulsified gel is a test with a laser scanning confocal microscope.

More specifically, labelling of the compositions by diffusion is carried out. For example, fluorochrome solutions are prepared, on the one hand starting from Fluorescein Salt (Sigma Aldrich) solubilized in water, in a proportion of [0.1 M], at ambient temperature, with gentle stirring, and on the other hand based on Nile Red (ThermoFisher) solubilized in a homosalate, octocrylene, ethylhexyl salicylate mixture [1; 0.7; 0.5] in a proportion of 240 ppm, at ambient temperature, with gentle stirring. 5 µl of each of the florochrome solutions are added to 1.5 ml of each of the compositions tested. The whole mixture is carefully mixed manually using a spatula and then left to stand for diffusion for a minimum of 3 h at ambient temperature. After diffusion of the fluorochrome solutions, each sample is placed between 2 microscope coverslips (thickness 170 µm) separated by a spacer of 250 µm. A laser scanning confocal microscope (TC SP8, Leica Microsystems) is used to carry out the observations. Two lasers (Argon - λ_{Ar} = 488 nm and He/Ne - λ_{He/Ne} = 561 nm) are used to excite respectively the fluorochromes Fluorescein (λ_{F - abs max} = 515 nm) and Nile Red (λ_{P - abs max} = 620 nm). Two PMT (PhotoMultiplier Tube) detectors are configured so as to recover the fluorescence emission signal respectively on the bandwidths Δλ_{F - em} = 506 - 546 nm and Δλ_{B - em} = 650 - 710 nm. The images are acquired in sequential mode, that is to say by alternating excitation and emission of the sample on 2 lasers. The hydrophilic and lipophilic phases resulting from the emission of fluorescence of the Fluorescein or the Nile Red appear in distinct colours. The microscopic observation of the compositions of gel/gel type shows a two-phase structure comprising a dispersion of lipophilic or hydrophilic domains having no specific or regular morphology (a first colour) in a hydrophilic or lipophilic continuous phase (in a second colour). The microscopic observation of the compositions of emulsion type shows a dispersion of lipophilic or hydrophilic spherical objects, in other words droplets (a first colour), in a hydrophilic or lipophilic continuous matrix (in a second colour). The microscopic observation of the compositions of aqueous emulsified gel type show a single hydrophilic continuous phase (in one colour).

In particular, the composition according to the invention contains the aqueous and oily phases in an aqueous phase/oily phase weight ratio ranging from 30/70 to 90/10, preferably from 60/40 to 80/20.

The ratio between the two phases is adjusted according to the desired cosmetic properties. Thus, in the case of a care composition, in particular for the face, it will be advantageous to favour an aqueous phase/oily phase weight ratio of greater than 1, notably ranging from 65/35 to 80/20, preferably ranging from 70/30 to 80/20.

These preferred ratios are particularly advantageous for obtaining fresh and light compositions.

Advantageously, a composition according to the invention may thus be in the form of a creamy gel with a minimum stress below which it does not flow unless it has been subjected to an external mechanical stress.

As emerges from the text hereinbelow, a composition according to the invention may have a minimum threshold stress of 1.5 Pa and in particular greater than 10 Pa.

It may also advantageously have a stiffness modulus G* at least equal to 400 Pa and preferably greater than 1000 Pa.

According to an advantageous embodiment variant, the phases under consideration for forming a composition according to the invention may have, respectively, a threshold stress of greater than 1.5 Pa and preferably greater than 10 Pa.

Characterization of the threshold stresses is performed by oscillating rheology measurements. Methodology is proposed in the illustrative chapter of the present text.

In general, the corresponding measurements are taken at 25°C using a Haake RS600 controlled-stress rheometer equipped with a cone-plate measuring body (60 mm diameter). For each measurement, the sample is placed delicately in position and the measurements start 5 minutes after placing the sample in the jaws (2 mm). The test composition is then subjected to a stress ramp from 10⁻² to 10³ Pa at a set frequency of 1 Hz.

A composition according to the invention may also have a certain elasticity. This elasticity may be characterized by a stiffness modulus G* which, under this minimum stress threshold, may be at least equal to 100 Pa and preferably greater than 400 Pa. The value G* of a composition may be obtained by subjecting the composition under consideration to a stress ramp from 10⁻² to 10³ Pa at a set frequency of 1 Hz.

### INTENDED USE OF THE COMPOSITION

The compositions, notably the non-therapeutic cosmetic compositions, according to the invention comprise a physiologically acceptable medium.

For the purposes of the present invention, the term "physiologically acceptable medium" is intended to denote a medium that is suitable for the topical administration of a composition. A physiologically acceptable medium generally has no unpleasant odour or appearance, and is entirely compatible with topical administration. In the present case, where the composition is intended for topical administration, i.e. by application at the surface of the keratin material under consideration, such a medium is considered in particular to be physiologically acceptable when it does not cause stinging, tautness or redness that is unacceptable to the user.

In particular, the composition is suited to topical application, that is to say application on the surface of the skin, the scalp and/or the mucous membrane under consideration. Thus, the physiologically acceptable medium is preferentially a cosmetically or dermatologically acceptable medium, i.e. a medium that has no unpleasant odour, colour or appearance, and that does not cause the user any unacceptable stinging, tautness or redness.

The composition according to the invention may be more or less fluid and may have the appearance of a white or coloured, preferably coloured, cream, an ointment, a milk, a lotion, a serum, a paste or a foam. It may optionally be applied to the skin in aerosol form. It may also be in solid form, for example in the form of a stick.

A composition according to the invention may be in the form of a non-therapeutic cosmetic composition for caring for and/or making up keratin materials, preferably a cosmetic composition for caring for keratin materials, in particular of the body or of the face, preferably of the face.

The compositions according to the invention may be in the form of products for caring for the skin or semi-mucous membranes, such as a protective or cosmetic care composition for the face, for the lips, for the hands, for the feet, for the anatomical folds or for the body (for example, day creams, night cream, day serum, night serum, makeup-removing cream, makeup base, antisun composition, protective or care body milk, aftersun milk, skincare or scalp-care lotion, gel or foam, serum, mask, or aftershave composition).

The cosmetic compositions according to the invention may be used, for example, as care products and/or antisun protection products for the face and/or the body, of liquid to semiliquid consistency, such as lotions, milks, more or less rich creams, gels and cream-gels. They may optionally be packaged in aerosol form and may be in the form of a mousse or a spray.

The composition may be applied to the skin by hand or using an applicator.

Such compositions are in particular prepared according to the general knowledge of those skilled in the art.

The compositions according to the invention in the form of vapourizable fluid lotions in accordance with the invention are applied to the skin or hair in the form of fine particles by means of pressurized or non-pressurized vapourizing devices. The devices in accordance with the invention are well known to those skilled in the art and comprise non-aerosol pumps or "atomizers", aerosol containers comprising a propellant and aerosol pumps using compressed air as propellant. The latter are described in patents US 4 077 441 and US 4 850 517.

The compositions packaged in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 80% by weight relative to the total weight of the composition.

Such compositions as defined previously may especially be employed for a non-therapeutic cosmetic use according to the invention.

The present disclosure also relates to the non-therapeutic cosmetic use of a composition according to the invention for caring for and/or making up keratin materials, preferably for caring for keratin materials, in particular the skin of the body and/or of the face.

The invention also relates to a non-therapeutic cosmetic process for making up and/or caring for keratin materials, in particular the skin, comprising at least one step of applying a composition as defined previously to said keratin materials.

Preferably, the invention also relates to a non-therapeutic cosmetic process for caring for keratin materials, in particular the skin, comprising at least one step of applying a composition as defined above to said keratin materials.

The present disclosure also relates to the non-therapeutic cosmetic use of a composition as described above, for protecting the skin and/or the lips and/or the hair against solar radiation.

The present disclosure further relates to the non-therapeutic cosmetic use of a composition as defined above, for combating or preventing the signs of photo-induced premature ageing of the skin and/or the lips and/or the hair.

According to one embodiment, the present disclosure also relates to a composition for use in reducing the pigmentation induced by long UVA rays and/or by exposure to DUV rays (UV with daily exposure or daily UV rays).

The present disclosure also relates to the non-therapeutic cosmetic use of a composition as defined above, for preventing darkening of the skin and/or improving the colour and/or the uniformity of the complexion.

In addition, the present disclosure also relates to the non-therapeutic cosmetic use of a composition as defined above, for preventing premature ageing of the skin, especially on the face, the neckline, the arms, the hands and/or the shoulders, in particular the signs of skin ageing of actinic origin, such as photoageing.

The present disclosure also relates to the non-therapeutic cosmetic use of a composition as defined above to prevent a loss of firmness and/or elasticity and/or tonicity and/or suppleness of the skin, the formation of wrinkles and fine lines, a dull complexion, and/or a wizened appearance of the skin.

The present disclosure also relates to a non-therapeutic cosmetic process for protecting the skin and/or the lips and/or the hair against solar radiation, comprising the application, to the surface of the keratin material, of at least one composition as defined above.

It also relates to a non-therapeutic cosmetic process for preventing and/or treating the signs of ageing of a keratin material, comprising the application, to the surface of the keratin material, of at least one composition as defined above.

For the purposes of the present invention, the term "preventing" is understood to mean, at least partly, reducing the risk of occurrence of a given phenomenon.

Throughout the description, including the claims, the expression "comprising a" should be understood as being synonymous with "comprising at least one", unless otherwise specified. The expressions "between... and...", "comprises from ... to...", "formed from ... to..." and "ranging from... to..." should be understood as being inclusive of the limits, unless otherwise specified.

The invention is illustrated in greater detail by the examples presented below. Unless otherwise indicated, the amounts shown are expressed as mass percentages.

### Example

### Measurement methods

In the examples detailed below, the following measurement methods are used.

### Methodology for the oscillating dynamic rheology measurements

These are harmonic-regime rheology measurements for measuring the elastic modulus. The measurements are taken using a Haake RS600 rheometer on a product at rest, at 25°C with a plate-plate rotor Ø 60 mm and a 2 mm gap.

The harmonic-regime measurements make it possible to characterize the viscoelastic properties of the products. The technique consists in subjecting a material to a stress which varies sinusoidally over time and in measuring the response of the material to this stress. In a range in which the behaviour is linear viscoelastic behaviour (zone in which the strain is proportional to the stress), the stress (τ) and the strain (γ) are two sinusoidal functions of time which are written in the following manner: τ(t) = τ₀ sin (ωt) and γ(t) = γ₀ sin (ωt + δ) where τ₀ represents the maximum amplitude of the stress (Pa); γ₀ represents the maximum amplitude of the strain (-); ω = 2ΠN represents the angular frequency (rad.s⁻¹) with N representing the frequency (Hz); and δ represents the phase shift of the stress relative to the strain (rad).

Thus, the two functions have the same angular frequency, but they are phase-shifted by an angle δ. Depending on the phase shift δ between τ(t) and γ(t), the behaviour of the system may be apprehended: if δ = 0, the material is purely elastic; if δ = Π/2, the material is purely viscous (Newtonian fluid); and if 0 < δ < Π/2, the material is viscoelastic.

In general, the stress and the strain are written in complex form: τ*(t) = τ₀ e^{iωt} and γ*(t) = γ₀ e^{(iωt + δ)}.

A complex stiffness modulus, representing the overall resistance of the material to the strain, whether it is of elastic or viscous origin, is then defined by G* = τ*/γ* = G' + iG" where G' is the storage modulus or elastic modulus, which characterizes the energy stored and totally restituted during a cycle, G' = (τ₀/ γ₀) cos δ; and G' is the loss modulus or viscous modulus, which characterizes the energy dissipated by internal friction during a cycle, G" = (τ₀/ γ₀) sin δ.

The parameter retained is the mean stiffness modulus G* recorded at the plateau measured at a frequency of 1 Hz.

### Protocol for measuring the stability

A method of evaluating the stability is carried out by observation of the composition over time, compared to a reference.

The instability can be detected by observation of a phase shift, of a release or of a modification of appearance.

Variations in texture, appearance, colour and odour may also be evaluated macroscopically (to the naked eye) and/or microscopically.

In particular, the compositions are observed after storage for 2 months at ambient temperature (20 - 25°C). For the purposes of comparison, the stability at two months at ambient temperature can be classified on a scale of 1 to 5, 1 corresponding to the least stable composition.

### Protocol for measuring the freshness

The freshness of the compositions is evaluated in a sensory manner on application, by a panel of 5 trained volunteers, on a scale of 1 to 5, 1 corresponding to the least fresh composition.

### Protocol for evaluating the transparency by measurement of l*

The measurement is carried out on a Turbiscan^{™} Lab Analyzer instrument from the company Formulaction. It consists in illuminating the sample and in measuring the transmitted intensity and the backscattered intensity by virtue of two synchronized detectors placed opposite and next to the light source.

The backscattering is directly connected to the average free path of the photons 1*, which is the distance from which the light photon loses its initial direction.

The value of 1* is given in µm. The higher this value, the more transparent the sample.

### Example I: Compositions according to the invention

The compositions (1) and (2) according to the invention, in gel/gel form, are prepared as described below with the proportions indicated in Table 1.

**Table 1**

| **Phase** | **Compounds** | **Composition 1 according to the invention** | **Composition 2 according to the invention** |
|---|---|---|---|
| A1 | 2-Ethylhexyl Salicylate (Ethylhexyl Salicylate) (*Parsol EHS*^{®}, *DSM Nutritional Products*) | 5 | 5 |
| | Pentane-1,2-Diol | 1.5 | 1.5 |
| A2 | Lauroylglutamic acid Dibutylamide (*GP-1*^{®}, *Ajinomoto*) | 1.5 | 1 |
| A3 | Poly(Stearyl Acrylate) (*Tego SP13-1*^{®}, *Evonik*) | 1.5 | 0.75 |
| | Homomenthyl salicylate (*Homosalate, Chemspec Chemicals*) | 5 | 5 |
| | 2- Ethylhexyl 2-Cyano-3,3-Diphenylacrylate (*Octocrylene Sunobel oct*^{®}, *Nanjing Cosmos Industria*) | 7 | 7 |
| | Dextrin palmitate (Rheopearl KL2 - OR^{®}, Chiba Flour Milling) | 1.5 | 0.9 |
| | 1-[4-(1,1-Dimethylethyl)Phenyl]-3-(4-Methoxyphenyl)Propane-1,3-Dione (Butyl Methoxydibenzoylmethane) (*Parsol 1789*^{®}, *DSM Nutritional Products*) | 5 | 5 |
| | 2-(2-H-Benxotriazole-2-yl)-4-Methyl-6-[2-Methyl-3-[1,3,3,3-Tetramethyl-1-[(Trumethylsilyl)Oxy]Disiloxanyl]Propyl]Phenol (Drometrizole Trisiloxane) (*Sunbellin*^{®}, *Shandong Keyuan Pharmaceutical*) | 1 | 1 |
| | Tris(2-Ethylhexyl)-4,4',4"-(1,3,5-Triazine-2,4,6-Triyltriimino)Tribenzoate (Ethylhexyl Triazone) (*Sunobel EHT*^{®}, *Nanjing Cosmos Industria*) | 1 | 1 |
| | Fragrance | / | 0.2 |
| | Vitamin E: DL-ALPHA-Tocopherol (*DL Alpha Tocopherol, DSM Nutritional Products*) | / | 0.2 |
| B1 | Glycerol | 7 | 7 |
| | Xanthan gum (*Keltrol CG*^{®}, *CP Kelco*) | 0.2 | 0.2 |
| B2 | Microbiologically clean deionized water | qs 100 | qs 100 |
| | Provitamin B5: Dexpanthenol (*D Panthenol Care*^{®}, *BASF*) | / | 0.1 |
| | Octane-1,2-Diol | 0.3 | 0.3 |
| | 2-Phenoxyethanol | 0.5 | 0.5 |
| | Disodium Salt of Brilliant Blue FCF (CI: 42090) (*Unicert Blue 05601-J*^{®}, *Sensient*) | / | 0.00036 |
| B3 | Ammonium Acryloyldimethyltaurate/VP Copolymer (*Aristoflex AVC*^{®}, *Clariant*) | 1.5 | 0.8 |
| | Sodium Hyaluronate (MW: 1 100 000) as powder | / | 0.1 |
| C | Mixture of Crosslinked Polydimethylsiloxane and of Polydimethylsiloxane (6 CST) (24/76) (*KSG 16*^{®}, *Shin Etsu*) | 2.5 | 2.5 |
| D | Denatured absolute Ethyl Alcohol | 4.5 | 4.5 |

### Preparation of the compositions

The compositions 1 and 2 are prepared according to the following procedure:
The components of phases A1 and A2 are heated until a transparent liquid is obtained.
The components of phase A3 are heated until a homogeneous yellow liquid is obtained.
The mixture of phases A1 and A2 is added to the hot phase A3.

Phases B1 to B3 are first mixed before being brought into contact with the previous oily mixture.

The components of phases C and D are then added.

### Results

Compositions 1 and 2 are stable.

In addition, they are fresh and transparent as soon as they are applied to the skin.

### Example II: Comparative compositions

The compositions (3), (4) and (5) outside the invention are prepared as described below with the proportions indicated in Table 2.

**Table 2**

| **Phase** | **Compounds** | **Composition 3 outside the invention** | **Composition 4 outside the invention** | **Composition 5 outside the invention** |
|---|---|---|---|---|
| A1 | 2-Ethylhexyl Salicylate (Ethylhexyl Salicylate) (*Parsol EHS*^{®}, *DSM Nutritional Products*) | 5 | 5 | 5 |
| | Pentane-1,2-Diol | 1.5 | 1.5 | 1.5 |
| A2 | Lauroylglutamic acid Dibutylamide (*GP-1*^{®}, *Ajinomoto*) | / | 0.75 | 1.125 |
| A3 | Poly(Stearyl Acrylate) (*Tego SP13-1*^{®}, *Evonik*) | 0.75 | 1.125 | 0.75 |
| | Homomenthyl salicylate (*Homosalate, Chemspec Chemicals*) | 5 | 5 | 5 |
| | 2- Ethylhexyl 2-Cyano-3,3-Diphenylacrylate (*Octocrylene Sunobel oct*^{®}, *Nanjing Cosmos Industria*) | 7 | 7 | 7 |
| | Dextrin palmitate (Rheopearl KL2 - OR^{®}, Chiba Flour Milling) | 0.75 | 0.75 | / |
| | 1-[4-(1,1-Dimethylethyl)Phenyl]-3-(4-Methoxyphenyl)Propane-1,3-Dione (Butyl Methoxydibenzoylmethane) (*Parsol 1789*^{®}, *DSM Nutritional Products*) | 5 | 5 | 5 |
| | 2-(2-H-Benxotriazole-2-yl)-4-Methyl-6-[2-Methyl-3-[1,3,3,3-Tetramethyl-1-[(Trumethylsilyl)Oxy]Disiloxanyl]Propyl]Phenol (Drometrizole Trisiloxane) (*Sunbellin*^{®}, *Shandong Keyuan Pharmaceutical*) | 1 | 1 | 1 |
| | Tris(2-Ethylhexyl)-4,4',4"-(1,3,5-Triazine-2,4,6-Triyltriimino)Tribenzoate (Ethylhexyl Triazone) (*Sunobel EHI*^{®}, *Nanjing Cosmos Industria)* | 1 | 1 | 1 |
| B1 | Glycerol | 7 | 7 | 7 |
| | Xanthan gum (*Keltrol CG*^{®}, *CP Kelco*) | 0.2 | 0.2 | 0.2 |
| B2 | Microbiologically clean deionized water | qs 100 | qs 100 | qs 100 |
| | Octane-1,2-Diol | 0.3 | 0.3 | 0.3 |
| | 2-Phenoxyethanol | 0.5 | 0.5 | 0.5 |
| B3 | Ammonium Acryloyldimethyltaurate/VP Copolymer (*Aristoflex AVC*^{®}, *Clariant*) | 0.75 | / | 0.75 |
| C | Mixture of Crosslinked Polydimethylsiloxane and of Polydimethylsiloxane (6 CST) (24/76) (*KSG 16*^{®}, *Shin Etsu)* | 2.5 | 2.5 | 2.5 |
| D | Denatured absolute Ethyl Alcohol | 4.5 | 4.5 | 4.5 |

### Preparation of the compositions

The compositions 3 to 5 are prepared according to the following procedure:
The components of phases A1 and A2 are heated until a transparent liquid is obtained.
The components of phase A3 are heated until a homogeneous yellow liquid is obtained.
The mixture of phases A1 and A2 is added to the hot phase A3.

Phases B1 to B3 are first mixed before being brought into contact with the previous oily mixture.

The components of phases C and D are then added.

### Results

Compared to the compositions 1 and 2, the compositions 3, 4 and 5 are less stable, less fresh and less transparent. The comparative data are shown in Table 3 below.

**Table 3**

| **Compositions** | **1 according to the invention** | **2 according to the invention** | **3 outside the invention** | **4 outside the invention** | **5 outside the invention** |
|---|---|---|---|---|---|
| Stability at 2 months at ambient temperature | 5 | 5 | 2 | 1 | 3 |
| Freshness | 5 | 5 | 2 | 2 | 4 |
| Transparency l*/mm | 3.00 | 3.00 | 0.65 | NA | 1.40 |

## Claims

1. Composition, notably cosmetic composition, in particular for making up and/or caring for keratin materials, comprising:
- at least one aqueous phase containing:
(i) at least a first hydrophilic gelling agent chosen from non-starchy polysaccharides; and
(ii) at least a second hydrophilic gelling agent chosen from 2-acrylamido-2-methylpropanesulfonic acid polymers and copolymers;
- at least one oily phase containing:
(a) at least a first lipophilic gelling agent chosen from amino acid-based gelling agents; and
(b) at least a second lipophilic gelling agent chosen from dextrin esters; and
- said composition also comprising at least one UV-screening agent.

2. Composition according to Claim 1, the first hydrophilic gelling agent (i) being present in a content ranging from 0.05% to 5% by weight, preferably ranging from 0.1% to 1% by weight, relative to the total weight of the composition.

3. Composition according to either one of the preceding claims, the first hydrophilic gelling agent (i) being chosen from carrageenans, in particular kappa carrageenan, gellan gum, agar-agar, xanthan gum, alginate-based compounds, in particular sodium alginate, scleroglucan gum, guar gum, inulin and pullulan, and mixtures thereof, and preferably the first hydrophilic gelling agent (i) being xanthan gum.

4. Composition according to any one of the preceding claims, the second hydrophilic gelling agent (ii) being present in a content ranging from 0.1% to 5% by weight, preferably ranging from 0.4% to 2% by weight, relative to the total weight of the composition.

5. Composition according to any one of the preceding claims, the second hydrophilic gelling agent (ii) being chosen from 2-acrylamido-2-methylpropanesulfonic acid copolymers, preferably from copolymers of 2-acrylamido-2-methylpropanesulfonic acid and of vinylpyrrolidone or of vinylformamide, and more preferentially from copolymers of 2-acrylamido-2-methylpropanesulfonic acid and of vinylpyrrolidone.

6. Composition according to any one of the preceding claims, the first lipophilic gelling agent (a) being present in a content ranging from 0.05% to 5% by weight, and preferably ranging from 0.1% to 1% by weight, relative to the total weight of the composition.

7. Composition according to any one of the preceding claims, the first lipophilic gelling agent (a) being chosen from dibutyl lauroyl glutamide and dibutyl ethylhexanoyl glutamide, and preferably being dibutyl lauroyl glutamide.

8. Composition according to any one of the preceding claims, the second lipophilic gelling agent (b) being present in a content ranging from 0.1% to 5% by weight, and preferably ranging from 0.4% to 2% by weight, relative to the total weight of the composition.

9. Composition according to any one of the preceding claims, the second lipophilic gelling agent (b) being chosen from esters of dextrin and of a C₁₂-C₁₈ fatty acid, in particular from esters of dextrin and of a C₁₄-C₁₈ fatty acid, preferably from dextrin myristate and/or dextrin palmitate, and more preferentially the second lipophilic gelling agent (b) being dextrin palmitate.

10. Composition according to any one of the preceding claims, also comprising at least a third lipophilic gelling agent (c), preferably chosen from (C₁₀-C₃₀)alkyl polyacrylates, and more preferentially chosen from stearyl polyacrylates.

11. Composition according to any one of the preceding claims, the third lipophilic gelling agent (c) being present in a content ranging from 0.2% to 5% by weight, and preferably ranging from 0.5% to 2% by weight, relative to the total weight of the composition.

12. Composition according to any one of the preceding claims, containing the aqueous and oily phases in an aqueous phase/oily phase weight ratio ranging from 30/70 to 90/10 and preferably from 60/40 to 80/20.

13. Compositions according to any one of the preceding claims, the UV-screening agent(s) being chosen from lipophilic UV-screening agents, and preferably from Butyl Methoxydibenzoylmethane, Homosalate, Ethylhexyl Salicylate, Octocrylene, Ethylhexyl triazone, Drometrizole Trisiloxane and mixtures thereof.

14. Composition according to any one of the preceding claims, the UV-screening agent(s) being present totally or partially, and preferably solely, in the oily phase.

15. Composition according to any one of the preceding claims, also comprising at least one alcohol, in particular a monoalcohol, and preferably ethanol.

16. Composition according to the preceding claim, the alcohol being present in a content ranging from 0.1% to 10% by weight, preferably ranging from 2% to 7% by weight, relative to the total weight of the composition.

17. Composition according to any one of the preceding claims, also comprising at least one additional cosmetic active agent, preferably hyaluronic acid, or a salt thereof, and more preferentially sodium hyaluronate.

18. Non therapeutic cosmetic process for making up and/or caring for a keratin material, in particular the skin and/or the lips, comprising at least one step which consists in applying to said keratin material a composition as defined according to any one of Claims 1 to 17.

## Patentansprüche

1. Zusammensetzung, insbesondere kosmetische Zusammensetzung, insbesondere zum Schminken und/oder Pflegen von Keratinmaterialien, umfassend:
- mindestens eine wässrige Phase enthaltend:
(i) mindestens ein erstes hydrophiles Geliermittel, das aus nicht stärkehaltigen Polysacchariden ausgewählt ist, und
(ii) mindestens ein zweites hydrophiles Geliermittel, das aus 2-Acrylamido-2-methylpropansulfonsäure-Polymeren und -Copolymeren ausgewählt ist;
- mindestens eine ölhaltige Phase enthaltend:
(a) mindestens ein erstes lipophiles Geliermittel, ausgewählt aus Geliermitteln auf Aminosäurebasis, und
(b) mindestens ein zweites lipophiles Geliermittel, ausgewählt aus Dextrinestern; und
- wobei die Zusammensetzung auch mindestens ein UV-Schutzmittel umfasst.

2. Zusammensetzung nach Anspruch 1, wobei das erste hydrophile Geliermittel (i) in einem Anteil von 0,05 bis 5 Gew.-%, vorzugsweise von 0,1 bis 1 Gew.-%, bezogen auf das Gesamtgewicht der Zusammensetzung, vorliegt.

3. Zusammensetzung nach einem der vorhergehenden Ansprüche, wobei das erste hydrophile Geliermittel (i) ausgewählt ist aus Carrageenanen, insbesondere Kappa-Carrageenan, Gellan-Gummi, Agar-Agar, Xanthan-Gummi, Verbindungen auf Alginat-Basis, insbesondere Natriumalginat, Skleroglucan-Gummi, Guar-Gummi, Inulin und Pullulan, und deren Mischungen, und wobei das erste hydrophile Geliermittel (i) vorzugsweise Xanthan-Gummi ist.

4. Zusammensetzung nach einem der vorhergehenden Ansprüche, wobei das zweite hydrophile Geliermittel (ii) in einem Anteil von 0,1 bis 5 Gew.-%, vorzugsweise von 0,4 bis 2 Gew.-%, bezogen auf das Gesamtgewicht der Zusammensetzung, vorhanden ist.

5. Zusammensetzung nach einem der vorhergehenden Ansprüche, wobei das zweite hydrophile Geliermittel (ii) ausgewählt ist aus 2-Acrylamido-2-methylpropansulfonsäure-Copolymeren, vorzugsweise aus Copolymeren von 2-Acrylamido-2-methylpropansulfonsäure und Vinylpyrrolidon oder Vinylformamid, und noch bevorzugter aus Copolymeren von 2-Acrylamido-2-methylpropansulfonsäure und Vinylpyrrolidon.

6. Zusammensetzung nach einem der vorhergehenden Ansprüche, wobei das erste lipophile Geliermittel (a) in einem Anteil von 0,05 bis 5 Gew.-% und vorzugsweise von 0,1 bis 1 Gew.- %, bezogen auf das Gesamtgewicht der Zusammensetzung, vorhanden ist.

7. Zusammensetzung nach einem der vorhergehenden Ansprüche, wobei das erste lipophile Geliermittel (a) aus Dibutyllauroylglutamid und Dibutylethylhexanoylglutamid ausgewählt ist und vorzugsweise Dibutyllauroylglutamid ist.

8. Zusammensetzung nach einem der vorhergehenden Ansprüche, wobei das zweite lipophile Geliermittel (b) in einem Anteil von 0,1 bis 5 Gew.-% und vorzugsweise von 0,4 bis 2 Gew.- %, bezogen auf das Gesamtgewicht der Zusammensetzung, vorhanden ist.

9. Zusammensetzung nach einem der vorhergehenden Ansprüche, wobei das zweite lipophile Geliermittel (b) ausgewählt ist aus den Estern von Dextrin und einer C₁₂ -C₁₈ Fettsäure, insbesondere aus den Estern von Dextrin und einer C₁₄ -C₁₈ Fettsäure, vorzugsweise aus Dextrinmyristat und/oder Dextrinpalmitat, und noch bevorzugter das zweite lipophile Geliermittel (b) Dextrinpalmitat ist.

10. Zusammensetzung nach einem der vorhergehenden Ansprüche, die auch mindestens ein drittes lipophiles Geliermittel (c) enthält, das vorzugsweise aus (C₁₀ -C₃₀)Alkylpolyacrylaten und noch bevorzugter aus Stearylpolyacrylaten ausgewählt ist.

11. Zusammensetzung nach einem der vorhergehenden Ansprüche, wobei das dritte lipophile Geliermittel (c) in einem Anteil von 0,2 bis 5 Gew.-% und vorzugsweise von 0,5 bis 2 Gew.- %, bezogen auf das Gesamtgewicht der Zusammensetzung, vorhanden ist.

12. Zusammensetzung nach einem der vorhergehenden Ansprüche, die die wässrigen und öligen Phasen in einem Gewichtsverhältnis von wässriger Phase/öliger Phase von 30/70 bis 90/10 und vorzugsweise von 60/40 bis 80/20 enthält.

13. Zusammensetzungen nach einem der vorhergehenden Ansprüche, wobei das/die UV-Schutzmittel aus lipophilen UV-Schutzmitteln und vorzugsweise aus Butylmethoxydibenzoylmethan, Homosalat, Ethylhexylsalicylat, Octocrylen, Ethylhexyltriazon, Drometrizoltrisiloxan und deren Mischungen ausgewählt sind.

14. Zusammensetzung nach einem der vorhergehenden Ansprüche, wobei das/die UV-Schutzmittel ganz oder teilweise, vorzugsweise ausschließlich, in der öligen Phase vorhanden ist/sind.

15. Zusammensetzung nach einem der vorhergehenden Ansprüche, die ferner mindestens einen Alkohol, insbesondere einen Monoalkohol, vorzugsweise Ethanol, umfasst.

16. Zusammensetzung nach dem vorhergehenden Anspruch, wobei der Alkohol in einem Anteil von 0,1 bis 10 Gew.-%, vorzugsweise von 2 bis 7 Gew.-%, bezogen auf das Gesamtgewicht der Zusammensetzung, vorhanden ist.

17. Zusammensetzung nach einem der vorhergehenden Ansprüche, die ferner mindestens einen zusätzlichen kosmetischen Wirkstoff, vorzugsweise Hyaluronsäure oder ein Salz davon, und noch bevorzugter Natriumhyaluronat, enthält.

18. Nichttherapeutisches kosmetisches Verfahren zum Schminken und/oder Pflegen eines Keratinmaterials, insbesondere der Haut und/oder der Lippen, das mindestens einen Schritt umfasst, der darin besteht, auf das Keratinmaterial eine Zusammensetzung gemäß einem der Ansprüche 1 bis 17 aufzutragen.

## Revendications

1. Composition, notamment composition cosmétique, en particulier pour le maquillage et/ou le soin de matières kératiniques, comprenant :
- au moins une phase aqueuse contenant :
(i) au moins un premier agent gélifiant hydrophile choisi parmi les polysaccharides non amylacés ; et
(ii) au moins un deuxième agent gélifiant hydrophile choisi parmi les polymères et les copolymères de l'acide 2-acrylamido-2-méthylpropanesulfonique ;
- au moins une phase huileuse contenant :
(a) au moins un premier gélifiant lipophile choisi parmi les agents gélifiants à base d'acides aminés ; et
(b) au moins un deuxième agent gélifiant lipophile choisi parmi les esters de dextrine ; et
- ladite composition comprenant en outre au moins un filtre UV.

2. Composition selon la revendication 1, le premier agent gélifiant hydrophile (i) étant présent en une teneur comprise dans la plage allant de 0,05 % à 5 % en poids, de préférence comprise dans la plage allant de 0,1 % à 1 % en poids, par rapport au poids total de la composition.

3. Composition selon l'une ou l'autre des revendications précédentes, le premier agent gélifiant hydrophile (i) étant choisi parmi les carraghénanes, notamment le carraghénane kappa, la gomme gellane, l'agar-agar, la gomme xanthane, les composés à base d'alginate, en particulier l'alginate de sodium, la gomme de scléroglucane, la gomme de guar, l'inuline et le pullulane, et des mélanges de ceux-ci, et de préférence le premier agent gélifiant hydrophile (i) étant la gomme xanthane.

4. Composition selon l'une quelconque des revendications précédentes, le deuxième agent gélifiant hydrophile (ii) étant présent en une teneur comprise dans la plage allant de 0,1 % à 5 % en poids, de préférence comprise dans la plage allant de 0,4 % à 2 % en poids, par rapport au total poids de la composition.

5. Composition selon l'une quelconque des revendications précédentes, le deuxième agent gélifiant hydrophile (ii) étant choisi parmi les copolymères de l'acide 2-acrylamido-2-méthylpropanesulfonique, de préférence parmi les copolymères de l'acide 2-acrylamido-2-méthylpropanesulfonique et de la vinylpyrrolidone ou du vinylformamide, et plus préférentiellement parmi les copolymères de l'acide 2-acrylamido-2-méthylpropanesulfonique et de la vinylpyrrolidone.

6. Composition selon l'une quelconque des revendications précédentes, le premier agent gélifiant lipophile (a) étant présent en une teneur comprise dans la plage allant de 0,05 % à 5 % en poids, et de préférence comprise dans la plage allant de 0,1 % à 1 % en poids, par rapport au poids total de la composition.

7. Composition selon l'une quelconque des revendications précédentes, le premier agent gélifiant lipophile (a) étant choisi parmi le dibutyllauroylglutamide et le dibutyléthylhexanoylglutamide, et de préférence étant le dibutyllauroylglutamide.

8. Composition selon l'une quelconque des revendications précédentes, le deuxième agent gélifiant lipophile (b) étant présent en une teneur comprise dans la plage allant de 0,1 % à 5 % en poids, et de préférence comprise dans la plage allant de 0,4 % à 2 % en poids, par rapport au poids total de la composition.

9. Composition selon l'une quelconque des revendications précédentes, le deuxième agent gélifiant lipophile (b) étant choisi parmi les esters de dextrine et d'un acide gras en C₁₂ à C₁₈, en particulier parmi les esters de dextrine et d'un acide gras en C₁₄ à C₁₈, de préférence parmi le myristate de dextrine et/ou le palmitate de dextrine, et plus préférentiellement le deuxième agent gélifiant lipophile (b) étant le palmitate de dextrine.

10. Composition selon l'une quelconque des revendications précédentes, comprenant en outre au moins un troisième agent gélifiant lipophile (c), de préférence choisi parmi les polyacrylates d'alkyle en C₁₀ à C₃₀, et plus préférentiellement choisi parmi les polyacrylates de stéaryle.

11. Composition selon l'une quelconque des revendications précédentes, le troisième agent gélifiant lipophile (c) étant présent en une teneur comprise dans la plage allant de 0,2 % à 5 % en poids, et de préférence comprise dans la plage allant de 0,5 % à 2 % en poids, par rapport au poids total de la composition.

12. Composition selon l'une quelconque des revendications précédentes, contenant les phases aqueuse et huileuse selon un rapport pondéral phase aqueuse/phase huileuse compris dans la plage allant de 30/70 à 90/10 et de préférence de 60/40 à 80/20.

13. Compositions selon l'une quelconque des revendications précédentes, le ou les filtres UV étant choisis parmi les filtres UV lipophiles, et de préférence parmi le butylméthoxydibenzoylméthane, l'homosalate, le salicylate d'éthylhexyle, l'octocrylène, l'éthylhexyltriazone, le drométrizole trisiloxane et des mélanges de ceux-ci.

14. Composition selon l'une quelconque des revendications précédentes, le ou les filtres UV étant présents totalement ou partiellement, et de préférence uniquement, dans la phase huileuse.

15. Composition selon l'une quelconque des revendications précédentes, comprenant en outre au moins un alcool, en particulier un monoalcool, et de préférence l'éthanol.

16. Composition selon la revendication précédente, l'alcool étant présent en une teneur comprise dans la plage allant de 0,1 % à 10 % en poids, de préférence comprise dans la plage allant de 2 % à 7 % en poids, par rapport au poids total de la composition.

17. Composition selon l'une quelconque des revendications précédentes, comprenant en outre au moins un agent actif cosmétique supplémentaire, de préférence l'acide hyaluronique, ou un sel de celui-ci, et plus préférentiellement le hyaluronate de sodium.

18. Procédé cosmétique non thérapeutique de maquillage et/ou de soin d'une matière kératinique, en particulier de la peau et/ou des lèvres, comprenant au moins une étape qui consiste à appliquer sur ladite matière kératinique une composition telle que définie selon l'une quelconque des revendications 1 à 17.
